(19) Europäisches Patentamt
European Patent Office
Office européen des brevets

(11) **EP 1 395 582 B1**

(12) **FASCICULE DE BREVET EUROPEEN**

(45) Date de publication et mention de la délivrance du brevet:
**04.05.2005 Bulletin 2005/18**

(21) Numéro de dépôt: **02738257.1**

(22) Date de dépôt: **17.05.2002**

(51) Int Cl.[7]: **C07D 413/06**, A61K 31/5377, A61P 11/00, A61P 25/00

(86) Numéro de dépôt international:
**PCT/FR2002/001663**

(87) Numéro de publication internationale:
**WO 2002/094821 (28.11.2002 Gazette 2002/48)**

(54) **NOUVEAUX DERIVES DE PIPERIDINECARBOXAMIDE, UN PROCEDE POUR LEUR PREPARATION ET LES COMPOSITIONS PHARMACEUTIQUES LES CONTENANT**

PIPERIDINCARBOXAMID-DERIVATE, EIN VERFAHREN ZU IHRER HERSTELLUNG UND SIE ENTHALTENDE ZUSAMMENSETZUNGEN

NOVEL PIPERIDINECARBOXAMIDE DERIVATIVES, METHOD FOR PREPARING SAME AND PHARMACEUTICAL COMPOSITIONS CONTAINING SAME

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **21.05.2001 FR 0106691**

(43) Date de publication de la demande:
**10.03.2004 Bulletin 2004/11**

(73) Titulaire: **Sanofi-Aventis**
**75013 Paris (FR)**

(72) Inventeurs:
- **EMONDS-ALT, Xavier**
**F-34980 Combaillaux (FR)**
- **PROIETTO, Vincenzo**
**F-34680 Saint Georges D'Orques (FR)**

(74) Mandataire: **Varady, Peter et al**
**Sanofi-Aventis**
**174 avenue de France**
**75013 Paris (FR)**

(56) Documents cités:
**EP-A- 1 048 658**
**WO-A-00/34274**
**WO-A-97/10211**
**FR-A- 2 791 346**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 395 582 B1

## Description

La présente invention a pour objet de nouveaux dérivés de pipéridinecarboxamide, un procédé pour leur préparation et les compositions pharmaceutiques en contenant en tant que principe actif.

Plus particulièrement, la présente invention concerne de nouveaux dérivés de pipéridinecarboxamide à usage thérapeutique, dans les phénomènes pathologiques qui impliquent le système des tachykinines comme par exemple de manière non limitative : la douleur (L. Urban et al., TINS, 1994, 17, 432-438 ; L. Seguin et al., Pain, 1995, 61, 325-343 ; S. H. Buck, 1994, The Tachykinin Receptors, Humana Press, Totowa, New-Jersey), l'allergie et l'inflammation (S. H. Buck, 1994, The Tachykinin Receptors, Humana Press, Totowa, New-Jersey), les troubles gastro-intestinaux (P. Holzer and U. Holzer-Petsche, Phamacol. Ther., 1997, 73, 173-217 et 219-263), les troubles respiratoires (J. Mizrahi et al., Pharmacology, 1982, 25, 39-50 ; C. Advenier et al., Eur. Respir. J., 1997, 10, 1892-1906 ; C. Advenier and X. Emonds-Alt, Pulmonary Pharmacol., 1996, 9, 329-333), les troubles urinaires (S. H. Buck, 1994, The Tachykinin Receptors, Humana Press, Totowa, New-Jersey ; C. A. Maggi, Progress in Neurobiology, 1995, 45, 1-98), les troubles neurologiques, les troubles neuropsychiatriques (C. A. Maggi et al., J. Autonomic Pharmacol., 1993, 13, 23-93 ; M. Otsuka and K. Yoshioka, Physiol. Rev. 1993, 73, 229-308).

Dans les années récentes de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : $NK_1$, $NK_2$, $NK_3$. La substance P (SP) est le ligand endogène des récepteurs $NK_1$, la neurokinine A (NKA) celui des récepteurs $NK_2$ et la neurokinine B (NKB), celui des récepteurs $NK_3$.

Les récepteurs $NK_1$, $NK_2$, $NK_3$ ont été mis en évidence chez différentes espèces.

Une revue de C. A. Maggi et al. (J. Autonomic Pharmacol., 1993, 13, 23-93) et une revue de D. Regoli et al. (Pharmacol. Rev., 1994, 46, 551-599) font le point sur les récepteurs aux tachykinines et leurs antagonistes et exposent les études pharmacologiques et les applications en thérapeutique humaine.

De nombreux brevets ou demandes de brevet décrivent des composés actifs sur les récepteurs des tachykinines. Ainsi la demande internationale WO 96/23787 concerne les composés de formule :

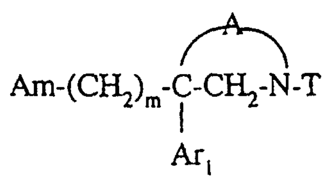

(A)

dans laquelle notamment :

- A peut représenter le radical bivalent $-O-CH_2-CH_2-$ ;
- Am, m, $Ar_1$ et T ont différentes valeurs.

En particulier le 1-[2-[4-benzoyl-2-(3,4-dichlorophényl)morpholin-2-yl]éthyl]-4-(pipéridin-1-yl)pipéridine-4-carboxamide (composé $\alpha$) est décrit à l'Exemple 65 de WO 96/23787.

Ce composé présente une forte affinité pour les récepteurs $NK_2$ humains mais une moins bonne affinité pour les récepteurs $NK_3$ humains.

La demande de brevet EP-A-0 776 893 concerne les composés de formule :

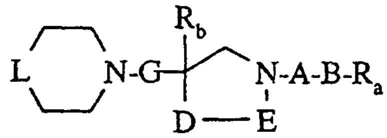

(B)

dans laquelle notamment :

- D-E peut représenter un radical bivalent $-O-CH_2-CH_2-$ ;
- L, G, E, A, B, $R_a$ et $R_b$ ont différentes valeurs.

La demande WO 00/34274 concerne des dérivés de cyclohexylpipéridine qui sont des antagonistes à la fois des récepteurs $NK_1$ de la substance P et des récepteurs $NK_2$ de la neurokinine A.

On a maintenant trouvé de nouveaux composés qui présentent une très forte affinité à la fois pour les récepteurs $NK_2$ humains de la neurokinine A et pour les récepteurs $NK_3$ humains de la neurokinine B et qui sont des antagonistes desdits récepteurs.

De plus, les composés selon la présente invention présentent une bonne biodisponibilité lorsqu'ils sont administrés par la voie orale.

Ces composés peuvent être utilisés pour la préparation de médicaments utiles dans le traitement de toute pathologie où soit la neurokinine A et/ou les récepteurs $NK_2$, soit la neurokinine B et/ou les récepteurs $NK_3$, soit à la fois la neurokinine A et la neurokinine B et/ou les récepteurs $NK_2$ et $NK_3$ sont impliqués, notamment dans le traitement des pathologies des systèmes respiratoire, gastro-intestinal, urinaire, immunitaire, cardiovasculaire, nerveux central ainsi que dans le traitement de la douleur, de la migraine, des inflammations, des nausées et des vomissements, des maladies de la peau.

Ainsi, selon un de ses aspects, la présente invention a pour objet des composés de formule :

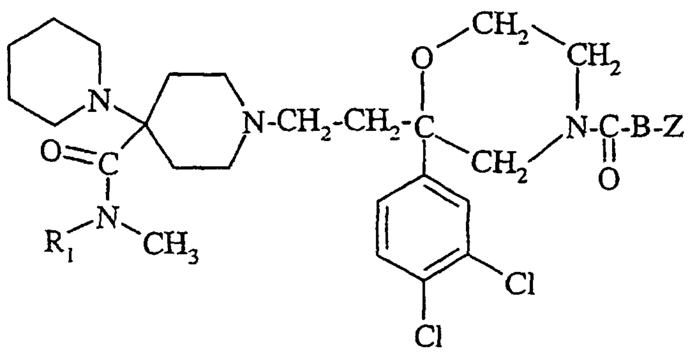

(I)

dans laquelle :

- $R_1$ représente un atome d'hydrogène ou un radical méthyle ;
- B représente une liaison directe ou un groupe $-CH_2-$ ;
- Z représente un phényle, un 2,3-dichlorophényle ou un 2,6-dichlorophényle ; ainsi que leurs sels avec des acides minéraux ou organiques, leurs solvats et/ou leurs hydrates.

Les composés de formule (I) selon l'invention comprennent aussi bien les isomères optiquement purs que leurs mélanges en proportions quelconques.

On peut former des sels des composés de formule (I). Ces sels comprennent aussi bien ceux avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels pharmaceutiquement acceptables, tels que le chlorhydrate, le bromhydrate, le sulfate, l'hydrogénosulfate, le dihydrogénophosphate, le méthanesulfonate, le méthylsulfate, l'oxalate, le maléate, le fumarate, le succinate, le naphtalène-2 sulfonate, le gluconate, le citrate, l'iséthionate, le benzènesulfonate, le paratoluènesulfonate, l'acétate.

Par atome d'halogène, on entend un atome de chlore, de brome, de fluor ou d'iode.

Selon la présente invention, on préfère les composés de formule (I) sous forme d'isomères optiquement purs.

Les composés suivants :

- N,N-diméthyl-1-[2-[4-benzoyl-2-(3,4-dichlorophényl) morpholin-2-yl]éthyl]-4-(pipéridin-1-yl)pipéridine-4-carboxamide, isomère dextrogyre ;
- N-méthyl-1-[2-[4-benzoyl-2-(3,4-dichlorophényl)morpholin-2-yl]éthyl]-4-(pipéridin-1-yl)pipéridine-4-carboxamide, isomère dextrogyre ;
- N,N-diméthyl-1-[2-[4-(2,3-dichlorobenzoyl)-2-(3,4-dichlorophényl)morpholin-2-yl]éthyl]-4-(pipéridin-1-yl)pipéridine-4-carboxamide, isomère lévogyre ;
- N,N-diméthyl-1-[2-[4-(2,6-dichlorophényl)acétyl]-2-(3,4-dichlorophényl)morpholin-2-yl]éthyl]-4-(pipéridin-1-yl)pipéridine-4-carboxamide, isomère dextrogyre ;
- N,N-diméthyl-1-[2-[4-[2-(2,3-dichlorophényl)acétyl]-2-(3,4-dichlorophényl)morpholin-2-yl]éthyl]-4-(pipéridin-1-yl) pipéridine-4-carboxamide, isomère dextrogyre ;

- N-méthyl-1-[2-[4-[2-(2,3-dichlorophényl)acétyl]-2-(3,4-dichlorophényl)morpholin-2-yl]éthyl]-4-(pipéridin-1-yl)pipéridine-4-carboxamide, isomère dextrogyre ;

ainsi que leurs sels avec des acides minéraux ou organiques, leurs solvats et/ou leurs hydrates sont préférés.

Le composé suivant :

- N,N-diméthyl-1-[2-[4-benzoyl-2-(3,4-dichlorophényl) morpholin-2-yl]éthyl]-4-(pipéridin-1-yl)pipéridine-4-carboxamide, isomère dextrogyre ;

ainsi que ses sels avec des acides minéraux ou organiques, ses solvats et/ou ses hydrates est particulièrement préféré.

Selon un autre de ses aspects, la présente invention concerne un procédé pour la préparation des composés de formule (I), de leurs sels, leurs solvats et/ou leurs hydrates, caractérisé en ce que :

on fait réagir un composé de formule :

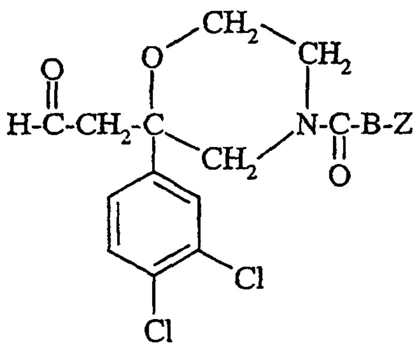

(II)

dans laquelle B et Z sont tels que définis pour un composé de formule (I), avec un composé de formule :

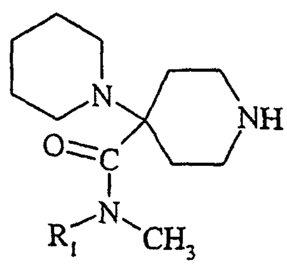

(III)

dans laquelle $R_1$ est tel que défini pour un composé de formule (I), en présence d'un acide, dans un solvant, puis on réduit le sel d'iminium formé intermédiairement au moyen d'un agent réducteur.

Eventuellement, on transforme le composé de formule (I) en l'un de ses sels avec des acides minéraux ou organiques.

La réaction s'effectue en présence d'un acide tel que l'acide acétique, dans un solvant tel que le méthanol ou le dichlorométhane, à une température comprise entre la température ambiante et la température de reflux du solvant, et forme *in-situ* une imine intermédiaire qui est réduite chimiquement en utilisant, par exemple, le cyanoborohydrure de sodium ou le triacétoxyborohydrure de sodium ou catalytiquement en utilisant l'hydrogène et un catalyseur tel que le palladium sur charbon ou le nickel de Raney®.

Selon une variante du procédé :

on fait réagir un composé de formule :

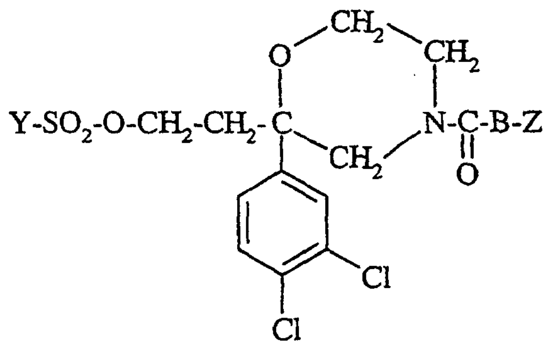

(IV)

dans laquelle B et Z sont tels que définis pour un composé de formule (I) et Y représente un groupe méthyle, phényle, tolyle ou trifluorométhyle, avec un composé de formule :

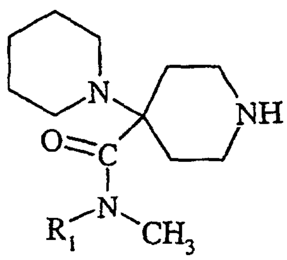

(III)

dans laquelle $R_1$ est tel que défini pour un composé de formule (I).

Eventuellement, on transforme le composé de formule (I) en un de ses sels avec des acides minéraux ou organiques.

La réaction s'effectue dans un solvant inerte tel que le N,N-diméthylformamide, l'acétonitrile, le chlorure de méthylène, le toluène ou l'isopropanol et en présence ou en l'absence d'une base. Lorsqu'on utilise une base, celle-ci est choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium. En l'absence de base, la réaction s'effectue en utilisant un excès du composé de formule (III) et en présence d'un iodure de métal alcalin tel que l'iodure de potassium ou l'iodure de sodium. La réaction s'effectue à une température comprise ente la température ambiante et 100°C.

Selon une autre variante du procédé
on fait réagir un composé de formule :

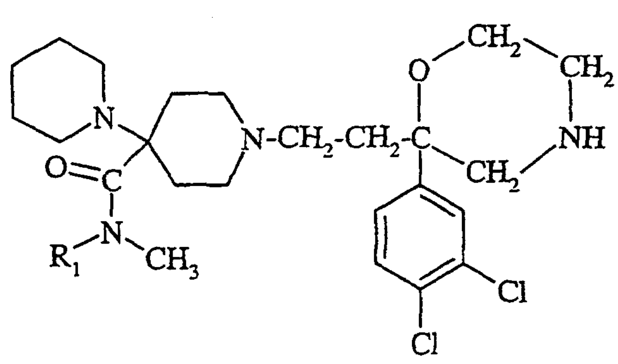

(V)

dans laquelle $R_1$ et tel que défini pour un composé de formule (I), avec un dérivé fonctionnel d'un acide de formule :

HOOC-B-Z (VI)

dans laquelle B et Z sont tels que définis pour un composé de formule (I).

Eventuellement, on transforme le composé de formule (I) en l'un de ses sels avec des acides minéraux ou organiques.

Comme dérivé fonctionnel de l'acide (VI), on utilise l'acide lui-même, ou bien un des dérivés fonctionnels qui réagissent avec les amines, par exemple un anhydride, un anhydride mixte, le chlorure d'acide, ou un ester activé, comme l'ester de paranitrophényle.

Lorsqu'on met en oeuvre l'acide de formule (VI) lui-même, on opère en présence d'un agent de couplage utilisé en chimie peptidique tel que le 1,3-dicyclohexyl carbodiimine ou l'hexafluorophosphate de benzotriazol-1-yloxytris(diméthylamino) phosphonium en présence d'une base telle que la triéthylamine ou la N,N-diisopropyl éthylamine, dans un solvant inerte tel que le dichlorométhane ou le N,N-diméthylformamide à une température comprise entre 0°C et la température ambiante.

Lorsqu'on utilise un chlorure d'acide, la réaction s'effectue dans un solvant inerte tel que le dichlorométhane ou le benzène, en présence d'une base telle que la triéthylamine ou la N-méthylmorpholine et à une température comprise entre -60°C et la température ambiante.

Les composés de formule (I) ainsi obtenus peuvent être ultérieurement séparés du milieu réactionnel et purifiés selon les méthodes classiques, par exemple par cristallisation ou chromatographie.

Les composés de formule (I) ainsi obtenus sont isolés sous forme de base libre ou de sel, selon les techniques classiques.

Lorsque les composés de formule (I) sont obtenus sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone ou dans le dichlorométhane, ou dans l'acétate d'éthyle ou dans l'acétonitrile avec une solution de l'acide choisi dans un des solvants précités, on obtient le sel correspondant qui est isolé selon les techniques classiques.

Ainsi, on prépare par exemple le chlorhydrate, le bromhydrate, le sulfate, le trifluoroacétate, l'hydrogénosulfate, le dihydrogénophosphate, le méthanesulfonate, l'oxalate, le maléate, le succinate, le fumarate, le naphtalène-2-sulfonate, le benzènesulfonate, le para-toluènesulfonate, le gluconate, le citrate, l'acétate.

A la fin de la réaction, les composés de formule (I) peuvent être isolés sous forme d'un de leurs sels, par exemple le chlorhydrate, ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les composés de formule (II) se préparent selon des méthodes connues telles que celles décrites dans WO 96/23787.

Par exemple, on prépare un composé de formule (II) selon le SCHEMA 1 ci-après dans lequel E représente un atome d'hydrogène ou un groupe O-protecteur.

SCHEMA 1

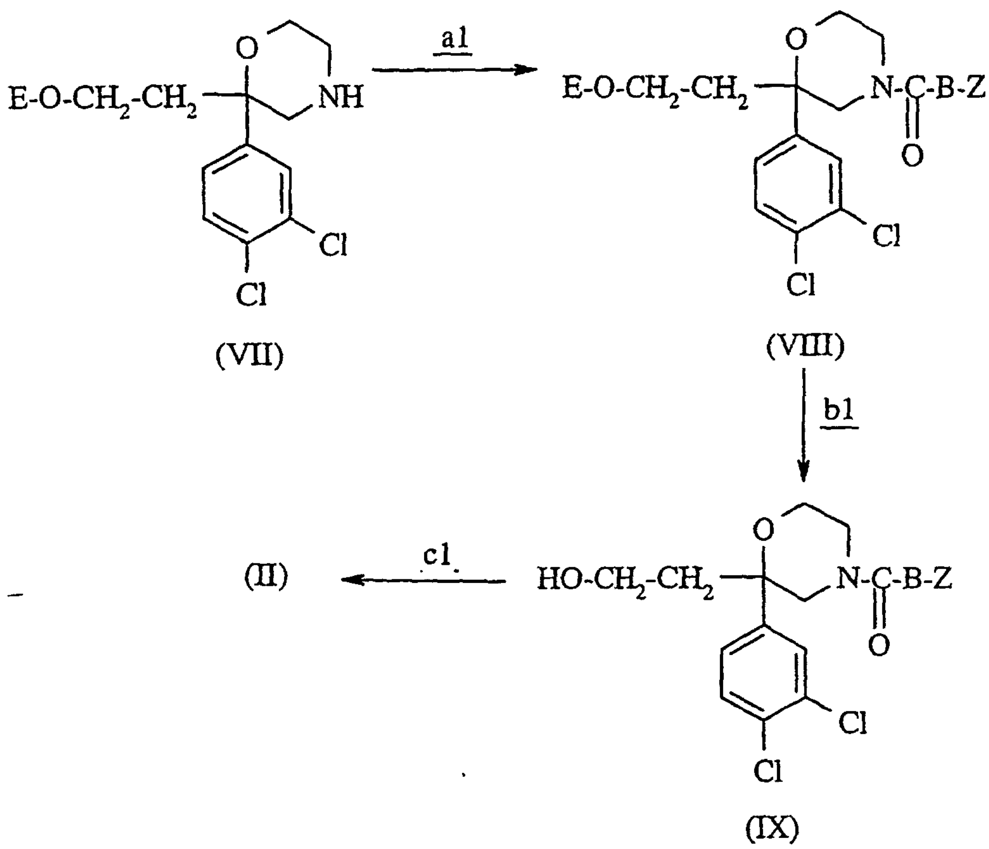


(VII)

(VIII)

(IX)

(II)

Lorsque E représente un groupe protecteur, celui-ci est choisi parmi les groupes O-protecteurs classiques bien connus de l'homme de l'art, tels que, par exemple le tétrahydropyran-2-yle, le benzoyle ou un ($C_1$-$C_4$)alkylcarbonyle.

A l'étape a1 du SCHEMA 1 on fait réagir un composé de formule (VII) avec un dérivé fonctionnel d'un acide de formule (VI), selon les méthodes précédemment décrites, pour obtenir un composé de formule (VIII).

Le composé de formule (VIII) ainsi obtenu est éventuellement déprotégé à l'étape b1 selon les méthodes connues de l'homme de l'art. Par exemple, lorsque E représente un groupe tétrahydropyran-2-yle, la déprotection s'effectue par hydrolyse acide en utilisant l'acide chlorhydrique dans un solvant tel que l'éther, le méthanol ou le mélange de ces solvants, ou en utilisant le *p*-toluènesulfonate de pyridinium dans un solvant tel que le méthanol ou encore, en utilisant une résine Amberlyst® dans un solvant tel que le méthanol. La réaction s'effectue à une température comprise entre la température ambiante et la température de reflux du solvant. Lorsque E représente un groupe benzoyle ou un groupe ($C_1$-$C_4$)alkylcarbonyle, la déprotection s'effectue par hydrolyse en milieu alcalin en utilisant par exemple un hydroxyde de métal alcalin tel que l'hydroxyde de sodium, l'hydroxyde de potassium ou l'hydroxyde de lithium, dans un solvant inerte tel que l'eau, le méthanol, l'éthanol, le dioxane ou un mélange de ces solvants, à une température comprise entre 0°C et la température de reflux du solvant.

A l'étape c1 on oxyde l'alcool de formule (IX) pour obtenir l'adéhyde de formule (II). La réaction d'oxydation s'effectue en utilisant par exemple le chlorure d'oxalyle, le diméthylsulfoxyde et la triéthylamine dans un solvant tel que le dichlorométhane et à une température comprise entre -78°C et la température ambiante.

Les composés de formule (III) sont connus et se préparent selon des méthodes connues. Par exemple, on prépare un composé de formule (III) selon le SCHEMA 2 ci-après.

SCHEMA 2

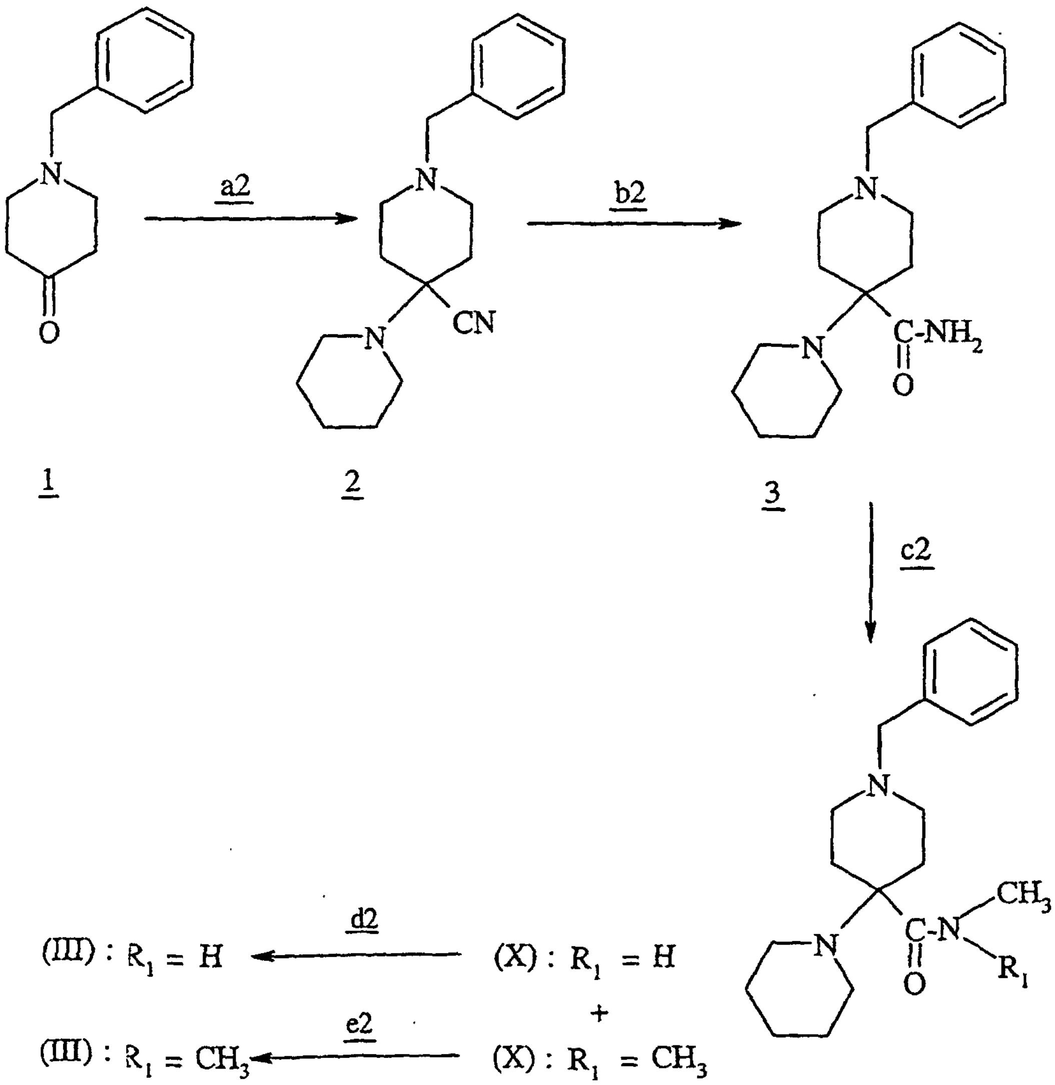


1 2 3

Les étapes a2 et b2 du SCHEMA 2 s'effectuent selon les modes opératoires décrits aux étapes A et B de la Préparation 2.16 dans WO 96/23787.

A l'étape c2, on fait réagir le composé 3 avec un halogénure de méthyle, de préférence l'iodure de méthyle, en présence d'une base forte telle que l'hydrure de sodium, dans un solvant tel que le tétrahydrofurane et à une température comprise entre la température ambiante et la température de reflux du solvant et obtient un mélange du composé de formule (X) dans laquelle $R_1$ = H et du composé de formule (X) dans laquelle $R_1$ = $CH_3$ que l'on sépare selon les méthodes classiques telles que par chromatographie.

Les composés (X) sont déprotégés aux étapes d2 ou e2 selon les méthodes connues pour donner les composés de formule (III) attendus.

Les composés de formule (IV) se préparent selon des méthodes connues telles que celles décrites dans WO 96/23787. Par exemple, on fait réagir un composé de formule (IX) avec un composé de formule :

$$Y\text{-}SO_2\text{-}Cl \quad (XI)$$

dans laquelle Y représente un groupe méthyle, phényle, tolyle ou trifluorométhyle. La réaction s'effectue en présence

d'une base telle que la triéthylamine, la pyridine, la N,N-diisopropylamine ou la N-méthylmorpholine, dans un solvant tel que le dichlorométhane ou le toluène, et à une température comprise entre -20°C et la température de reflux du solvant.

Les composés de formule (V) se préparent selon le SCHEMA 3 ci-après dans lequel E représente l'hydrogène ou un groupe O-protecteur et Pr représente un groupe N-protecteur.

SCHEMA 3

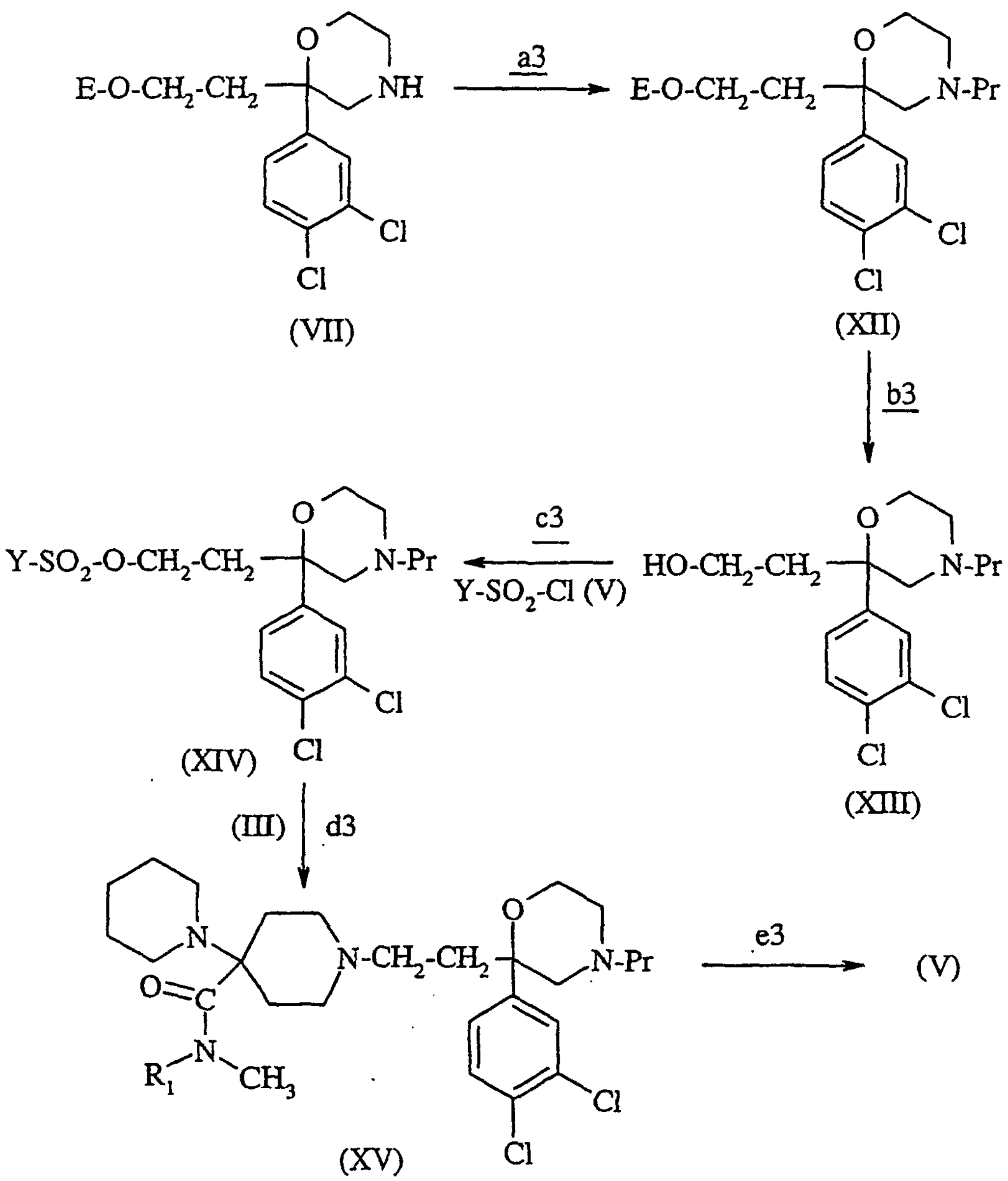


Lorsque Pr représente un groupe N-protecteur, celui-ci est choisi parmi les groupes N-protecteurs classiques bien connus de l'homme de l'art tels que, par exemple le groupe *tert*-butoxycarbonyle, le benzyloxycarbonyle ou le trityle.

Les composés de formule (VI) sont commerciaux ou préparés selon des méthodes connues. Ainsi, par exemple, l'acide 2-(2,3-dichlorophényl)acétique se prépare selon le SCHEMA 4 ci-après en suivant les modes opératoires décrits à la Préparation 1.1.

SCHEMA 4

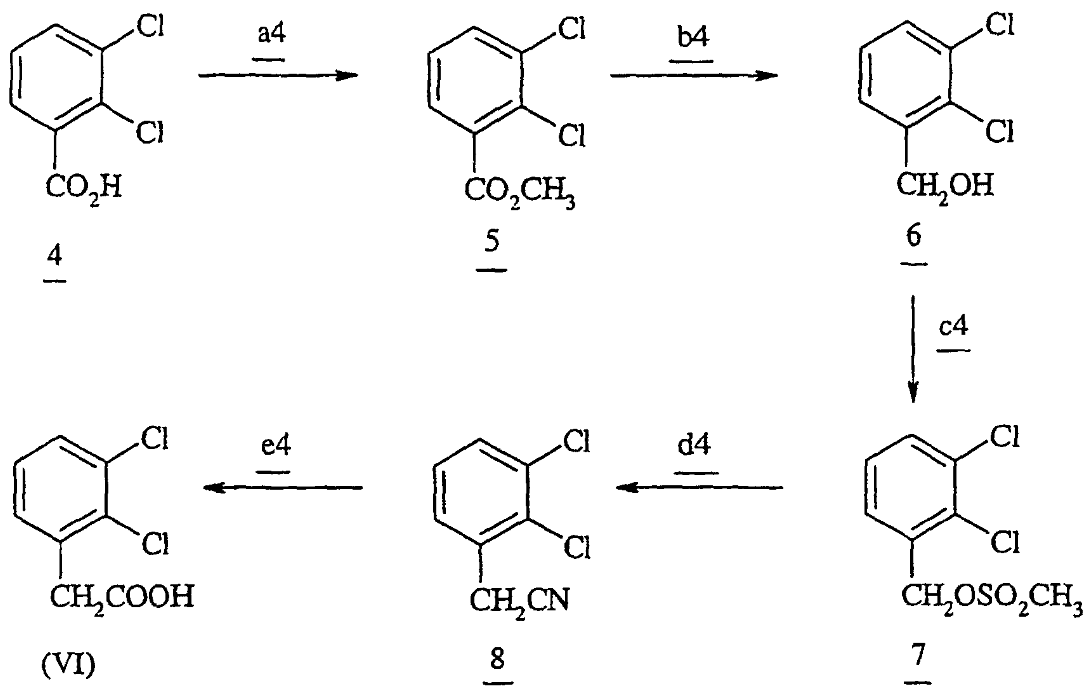


Les composés de formule (VII) sont connus et préparés selon des méthodes connues telles que celles décrites dans WO 96/23787, dans WO 01/04105, dans WO 00/58292 ou dans Tetrahedron : Asymmetry,1988, 9, 3251-3262.

Au cours de l'une quelconque des étapes de préparation des composés de formule (I) ou des composés intermédiaires de formule (II), (III), (IV), (V) ou (VI), il peut être nécessaire et/ou souhaitable de protéger les groupes fonctionnels réactifs ou sensibles, tels que les groupes amine, hydroxyle ou carboxy, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, ed. Plenum Press, 1973, dans Protective Groups in Organic Synthesis, T.W. Greene et P.G.M. Wutts, Ed. John Wiley et sons, 1991 ou dans Protecting Groups, Kocienski P.J., 1994, Georg Thieme Verlag. L'élimination des groupes protecteurs peut s'effectuer à une étape ultérieure opportune en utlisant les méthodes connues de l'homme de l'art et qui n'affectent pas le reste de la molécule concernée.

La résolution des mélanges racémiques des composés de formule (I) permet d'isoler les énantiomères.

Il est cependant préférable d'effectuer le dédoublement des mélanges racémiques à partir du composé de formule (VII, E = H) ou bien à partir d'un composé intermédiaire utile pour la préparation d'un composé de formule (VII), selon les méthodes décrites dans les publications précédemment citées pour la préparation d'un composé de formule (VII).

Les composés de formule (I) ci-dessus comprennent également ceux dans lesquels un ou plusieurs atomes d'hydrogène, ou de carbone ont été remplacés par leur isotope radioactif par exemple le tritium, ou le carbone-14. De tels composés marqués sont utiles dans des travaux de recherche, de métabolisme ou de pharmacocinétique, dans des essais biochimiques en tant que ligand de récepteurs.

Les composés selon l'invention ont fait l'objet d'essais biochimiques.

L'affinité des composés pour les récepteurs aux tachykinines a été évaluée in vitro par plusieurs essais biochimiques utilisant des radioligands :

1) La liaison de [$^{125}$I]BH-SP (Substance P marquée à l'iode-125 à l'aide du réactif de Bolton-Hunter) aux récepteurs $NK_1$ des cellules lymphoblastiques humaines (D. G. Payan et al., J. Immunol., 1984, 133, 3260-3265).
2) La liaison [$^{125}$I]His-NKA aux récepteurs clonés $NK_2$ humains exprimés par des cellules CHO (Y. Takeda et al., J. Neurochem., 1992, 59, 740-745).
3) La liaison [$^{125}$IHis[MePhe$^7$]NKB aux récepteurs clonés $NK_3$ humains exprimés par des cellules CHO (Buell et al., FEBS Letters, 1992, 299, 90-95).

Les essais ont été effectués selon X. Emonds-Alt et al. (Eur. J. Pharmacol., 1993, 250, 403-413 ; Life Sci., 1995, 56, PL 27-32).

Les composés selon l'invention inhibent faiblement la liaison de la substance P aux récepteurs $NK_1$ des cellules lymphoblastiques humaines IM9. La constante d'inhibition Ki pour les récepteurs des cellules lymphoblastiques humaines est supérieure ou égale à $8.10^{-9}$M.

Les composés selon l'invention inhibent fortement la liaison [$^{125}$I]His-NKA aux récepteurs clonés $NK_2$ humains. La constante d'inhibition Ki est inférieure ou égale à $5.10^{-10}$M. Ainsi, le composé de l'Exemple 1 possède un Ki égal à $4.10^{-11}$M.

Les composés selon l'invention inhibent fortement la liaison de [$^{125}$I]His[MePhe$^7$] NKB aux récepteurs clonés $NK_3$ humain : la constante d'inhibition Ki est inférieure ou égale à $7.10^{-10}$M. Ainsi, le composé de l'Exemple 1 possède un Ki égal à $4.10^{-11}$M.

Le composé $\alpha$ de l'art antérieur inhibe la liaison de [$^{125}$I]His-NKA aux récepteurs clonés $NK_2$ avec un Ki égal à $4.10^{-11}$M. Il inhibe la liaison de de [$^{125}$I]His[MePhe$^7$] NKB aux récepteurs clonés $NK_3$ humains avec un Ki égal à $2.10^{-9}$M.

Les composés de la présente invention ont également été évalués in vivo sur des modèles animaux.

Chez la gerbille, un comportement de rotation est induit par administration intrastriatale d'un agoniste spécifique du récepteur $NK_2$ la [Nle$^{10}$]NKA(4-10) ; on a constaté qu'une application unilatérale de [Nle$^{10}$]NKA(4-10) dans le striatum de gerbille conduit à de fortes rotations contralatérales qui sont inhibées par les composés selon l'invention administrés soit par voie intrapéritonéale, soit par voie orale. Ce test a été effectué selon M. Poncelet et al., Neurosci. Lett., 1993, 149, 40-42. Dans ce test, les composés selon l'invention sont actifs à des doses variant de 0,1 mg à 30 mg par kg. Par exemple, le composé de l'Exemple 1 possède une dose efficace 50 ($DE_{50}$) de 2,9 mg par kg par voie intrapéritonéale et une $DE_{50}$ de 6,5 mg par kg par voie orale.

Chez la gerbille, un comportement de rotation est induit par administration intrastriale d'un agoniste spécifique du récepteur $NK_3$ : le senktide ; on a constaté qu'une application unilatérale de senktide dans le striatum de gerbille conduit à de fortes rotations contralatérales qui sont inhibées par les composés selon l'invention administrés soit par voie intrapéritonéale, soit par voie orale. Ce test a été effectué selon X. Emonds-Alt et al., Life Sci., 1995, 56, PL27-PL32. Dans ce test, les composés selon l'invention sont actifs à des doses variant de 0,1 mg à 30 mg par kg. Par exemple, le composé de l'Exemple 1 possède une $DE_{50}$ de 2,8 mg par kg par voie intrapéritonéale et une $DE_{50}$ de 4,3 mg par kg par voie orale.

Chez le rat, l'application d'un agoniste des récepteurs $NK_2$ au niveau du septum provoque une augmentation de libération d'acétylcholine dans l'hippocampe (test effectué selon R. Steinberg et al., Eur. J. Neurosci., 1998, 10, 2337-2345). De même chez le cobaye, l'application locale d'un agoniste des récepteurs $NK_3$ au niveau du septum provoque une augmentation de libération d'acétylcholine dans l'hippocampe (test effectué selon N. Marco et al., Neuropeptides, 1998, 32, 481-488). Les composés selon l'invention bloquent cette augmentation de libération d'acétylcholine qu'elle soit provoquée par un agoniste des récepteurs $NK_2$ ou par un agoniste des récepteurs $NK_3$. Par exemple, le composé de l'Exemple 1 bloque cette augmentation de libération d'acétylcholine provoquée soit par un agoniste des récepteurs $NK_2$ chez le rat, soit par un agoniste des récepteurs $NK_3$ chez le cobaye, respectivement à des doses de 0,1-0,3 mg/kg et 0,3-1 mg/kg par voie intrapéritonéale.

Chez le rat, un stress de contrainte provoque une augmentation du taux tissulaire de DOPAC (de l'anglais, 3,4-dihydroxyphenyl acetic acid) dans le cortex préfrontal (test effectué selon B.A. Morrow et al., Eur. J. Pharmacol., 1993, 238, 255-262). Cette augmentation est bloquée par un antagoniste spécifique des récepteurs $NK_2$ tel que le saredutant (X. Emonds-Alt et al., Life Sci., 1992, 50, PL101-PL106), et par conséquent est médié par l'activation des récepteurs $NK_2$ par la neurokinine A endogène. On constate que, le composé de l'Exemple 1 administré à 1 mg/kg par voie intrapéritonéale bloque complètement cette augmentation.

Chez le cobaye, un traitement à l'halopéridol, administré à une dose de 1 mg/kg par voie intrapéritonéale, provoque une augmentation du nombre de neurones dopaminergiques spontanément actifs (réponse de population) dans la région A10 (VTA, de l'anglais ventral tegmental area) du cerveau, mesurée en électrophysiologie. Cette augmentation est médiée par l'activation des récepteurs $NK_3$ par la neurokinine B endogène (C. Gueudet et al., Synapse, 1999, 33, 71-79). On constate que, le composé de l'Exemple 1 administré à 0,1-1 mg/kg par voie intrapéritonéale bloque cette augmentation.

L'ensemble de ces résultats pharmacologiques montrent que les composés selon l'invention, en particulier le composé de l'exemple 1, sont des antagonistes mixtes des récepteurs $NK_2$ et des récepteurs $NK_3$ en bloquant les effets pharmacologiques provoqués par la neurokinine A ou la neurokinine B, qu'elles soient appliquées de manière exogène ou que leur libération endogène soit provoquée. De plus, ces résultats montrent que les composés selon l'invention passent bien la barrière hématoencéphalique.

Les composés de la présente invention sont notamment des principes actifs de compositions pharmaceutiques, dont la toxicité est compatible avec leur utilisation en tant que médicament.

Les composés de formule (I) ci-dessus peuvent être utilisés à des doses journalières de 0,01 à 100 mg par

kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Pour leur utilisation comme médicaments, les composés de formule (I) sont généralement administrés en unités de dosage. Lesdites unités de dosage sont de préférence formulées dans des compositions pharmaceutiques dans lesquelles le principe actif est mélangé avec un ou plusieurs excipients pharmaceutiques.

Ainsi, selon un autre de ses aspects, la présente invention concerne des compositions pharmaceutiques renfermant, en tant que principe actif, un composé de formule (I) ou l'un de ses sels, solvats et/ou hydrates pharmaceutiquement acceptables.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Lorsque l'on prépare une composition solide sous forme de comprimés ou de gélules, on ajoute au principe actif, micronisé ou non, un mélange d'excipients pharmaceutiques qui peut être composé de diluants comme par exemple le lactose, la cellulose microcristalline, l'amidon, le phosphate dicalcique, de liants comme par exemple la polyvinylpyrrolidone, l'hydroxypropylméthylcellulose, des délitants comme la polyvinylpyrrolidone réticulée, la carboxyméthylcellulose réticulée, des agents d'écoulement comme la silice, le talc, des lubrifiants comme le stéarate de magnésium, l'acide stéarique, le tribéhénate de glycérol, le stéarylfumarate de sodium.

Des agents mouillants ou tensioactifs tels que le laurylsulfate de sodium, le polysorbate 80, le poloxamer 188 peuvent être ajoutés à la formulation.

Les comprimés peuvent être réalisés par différentes techniques, compression directe, granulation sèche, granulation humide, fusion à chaud (hot-melt).

Les comprimés peuvent être nus ou dragéifiés (par du saccharose par exemple) ou enrobés avec divers polymères ou autres matières appropriés.

Les comprimés peuvent avoir une libération flash, retardée ou prolongée en réalisant des matrices polymériques ou en utilisant des polymères spécifiques au niveau du pelliculage.

Les gélules peuvent être molles ou dures, pelliculées ou non de manière à avoir une activité flash, prolongée ou retardée (par exemple par une forme entérique).

Elles peuvent contenir non seulement une formulation solide formulée comme précédemment pour les comprimés mais aussi des liquides ou des semi-solides.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un tensioactif hydrophile tel que le polysorbate 80 ou le poloxamer 188. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres, des sprays.

Pour l'administration transdermique, on peut utiliser des patches sous forme multilaminée ou à réservoir dans lesquels le principe actif peut être en solution alcoolique, des sprays.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane, des substituts des fréons ou tout autre gaz propulseur biologiquement compatible ; on peut également utiliser un système contenant le principe actif seul ou associé à un excipient, sous forme de poudre.

Le principe actif peut être également présenté sous forme de complexe avec une cyclodextrine, par exemple,

α, β, γ-cyclodextrine, 2-hydroxypropyl-β-cyclodextrine.

Le principe actif peut être formulé également sous forme de microcapsules ou microsphères, éventuellement avec un ou plusieurs supports ou additifs.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,1 à 1000 mg de principe actif, de préférence de 0,5 à 250 mg devant être administrés une à quatre fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Selon un autre de ses aspects, la présente invention concerne l'utilisation des composés de formule (I), ou de l'un de leurs sels, solvats et/ou hydrates pharmaceutiquement acceptables pour la préparation de médicaments destinés à traiter toute pathologie où soit la neurokinine A et/ou les récepteurs $NK_2$, soit la neurokinine B et/ou les récepteurs $NK_3$, soit à la fois la neurokinine A et la neurokinine B et/ou les récepteurs $NK_2$ et $NK_3$ sont impliqués.

Selon un autre de ses aspects, la présente invention concerne l'utilisation des composés de formule (I) ou de l'un de leurs sels, solvats et/ou hydrates pharmaceutiquement acceptables pour la préparation de médicaments destinés à traiter les pathologies du système respiratoire, gastro-intestinal, urinaire, immunitaire, cardiovasculaire et du système nerveux central ainsi que la douleur, la migraine, les inflammations, les nausées et les vomissements, les maladies de la peau.

Par exemple et de manière non limitative, les composés de formule (I) sont utiles :

- comme analgésique, en particulier dans le traitement de douleurs traumatiques telles que les douleurs post-opératoires ; de la névralgie du plexus brachial ; de douleurs chroniques telles que les douleurs arthritiques due à l'ostéoarthrite, l'arthrite rhumatoïde ou l'arthrite psoriatique ; de douleurs neuropathiques telles que la névralgie post-herpétique, la névralgie du trijumeau, la névralgie segmentale ou intercostale, la fibromyalgie, la causalgie, la neuropathie périphérique, la neuropathie diabétique, les neuropathies induites par une chimiothérapie, les neuropathies liées au SIDA, la névralgie occipitale, la névralgie géniculée, la névralgie glossopharyngienne ; de douleurs de l'illusion des amputés ; de diverses formes de mal de tête telle que la migraine chronique ou aiguë, de douleur temporomandibulaire, de douleur du sinus maxillaire, du névralgisme facial, d'odontalgie ; de douleur du cancéreux ; de douleur d'origine viscérale ; de douleur gastro-intestinale ; de douleur due à la compression nerveuse, de douleurs dues à la pratique intensive d'un sport ; de la dysménorrhée ; de douleur menstruelle ; de douleur due à une méningite, à une arachnoïdite ; de douleur muscosquelettique ; de douleurs du bas du dos dues à une sténose spinale, à un disque prolabé, à une sciatique ; de douleur de l'angineux ; de douleurs dues à une spondylite ankylosante ; de douleurs liées à la goutte ; de douleurs liées aux brûlures, à la cicatrisation, à une dermatose prurigineuse ; de douleur thalamique ;
- comme antiinflammatoire en particulier pour traiter les inflammations dans l'asthme, l'influenza, les bronchites chroniques (en particulier la bronchite chronique obstructive, COPD de l'anglais chronic obstructive pulmonary disease), les toux, les allergies, le bronchospasme et l'arthrite rhumatoïde ; les maladies inflammatoires du système gastro-intestinal par exemple la maladie de Crohn, les colites ulcératives, les pancréatites, les gastrites, l'inflammation des intestins, les troubles causés par les antiinflammatoires non-stéroidiens, les effets inflammatoires et secrétoires dûs aux infections bactériennes par exemple dûe au Clostridium difficile ; les maladies inflammatoires de la peau, par exemple l'herpès et l'eczéma ; les maladies inflammatoires de la vessie telles que la cystite et l'incontinence ; les inflammations ophtalmiques telles que la conjonctivite, la vitréorétinopathie ; les inflammations dentaires telles que gingivite et périodontite ;
- dans le traitement des maladies allergiques en particulier de la peau comme l'urticaire, les dermatites de contact, les dermatites atopiques et des maladies respiratoires comme les rhinites ;
- dans le traitement des maladies du système nerveux central en particulier des psychoses telles que la schizophrénie, la manie et la démence ; des désordres cognitifs telle que la maladie d'Alzheimer, l'anxiété, la démence liée au SIDA ; des neuropathies diabétiques ; de la dépression ; de la maladie de Parkinson ; de la dépendance aux drogues; des abus de substances ; des troubles de la vigilance, du sommeil, du rythme circadien, de l'humeur, de l'épilepsie ; du syndrôme de Down ; de la chorée d'Huntington ; des désordres somatiques liés au stress ; des maladies neurodégénératives telles que la maladie de Pick, la maladie de Creutzfeldt-Jacob ; des désordres liés à la panique, à la phobie, au stress ;

- dans le traitement des modifications de la perméabilité de la barrière hématoencéphalique durant les processus inflammatoires et auto-immuns du système nerveux central, par exemple lors d'infections liées au SIDA ;
- comme myorelaxant et antispasmodique ;
- dans le traitement des nausées et vomissements aigus ou retardés et anticipés, par exemple les nausées et vomissements induits par des drogues comme les agents utilisés en chimiothérapie lors d'un cancer ; par thérapies par rayons lors d'irradiations du thorax ou de l'abdomen dans le traitement du cancer, de carcinoïdose ; par ingestion de poison ; par des toxines dues à des désordres métaboliques ou infectieux comme les gastrites, ou produites lors d'infection gastro-intestinale bactérienne ou virale ; lors d'une grossesse ; lors de troubles vestibulaires telles que le mal des transports, les vertiges, le syndrome de Ménière ; lors des maladies post-opératoires ; les nausées et vomissements induits par dialyse, par les prostaglandines ; par obstructions gastro-intestinales ; lors de motilité gastro-intestinale réduite ; lors de douleurs viscérales par infarctus du myocarde ou péritonite ; lors de migraine ; lors du mal d'altitude ; par ingestion d'analgésiques opiacés comme la morphine ; lors de reflux gastro-oesophagien ; lors d'indigestion acide ou de surconsommation de nourriture ou de boisson, lors d'acidité ou aigreur gastrique, régurgitation, brûlure d'estomac, par exemple épisodiques, nocturnes ou induites par un repas et dyspepsie ;
- dans le traitement des maladies du système gastro-intestinal telles que le syndrome du colon irritable, les ulcères gastriques et duodénaux, les ulcères oesophagiques, les diarrhées, les hypersécrétions, les lymphomes, les gastrites, les reflux gastro-oesophagiens, l'incontinence fécale, la maladie de Hirschsprung ;
- dans le traitement des maladies de la peau telles que le psoriasis, les prurits, les brûlures, notamment les coups de soleil ;
- dans le traitement des maladies du système cardiovasculaire telles que l'hypertension, les aspects vasculaires de la migraine, les oedèmes, la thrombose, l'angine de poitrine, les spasmes vasculaires, les maladies circulatoires dûes à une vasodilatation, la maladie de Raynaud, les fibroses, les maladies du collagène, l'athérosclérose, la préeclampsie ;
- dans le traitement des cancers du poumon à petites cellules et à grandes cellules ; des cancers du sein ; des tumeurs cérébrales ; des adéno-carcinomes de la sphère urogénitale ; dans le traitement adjuvant pour prévenir les métastases ;
- les maladies de démyelination telles que la sclérose en plaques ou la sclérose latérale amyotrophique ;
- dans le traitement des maladies du système immunitaire liées à la suppression ou à la stimulation des fonctions des cellules immunes par exemple l'arthrite rhumatoïde, le psoriasis, la maladie de Crohn, le diabète, le lupus, les réactions de rejet après transplantation ;
- dans le traitement des troubles de la miction en particulier la pollakiurie, l'incontinence d'effort, l'incontinence d'urgence, l'incontinence post-partum ;
- dans le traitement de la réticulose histiocytaire comme dans les tissus lymphatiques ;
- comme anorexigène ;
- dans le traitement de l'emphysème ; du syndrome de Reiter ; des hémorroïdes ;
- dans le traitement des troubles oculaires telles que le glaucome, l'hypertension oculaire, le myosis, l'excès de larmes ;
- dans le traitement ou la prévention d'une attaque, de l'épilepsie, des traumatismes de la tête, des traumatismes du cordon spinal, des lésions ischémiques cérébrales dûe à une attaque ou à une occlusion vasculaire ;
- dans le traitement des troubles de la fréquence et du rythme cardiaque, en particulier ceux qui sont occasionnés par la douleur ou le stress ;
- dans le traitement des peaux sensibles et pour prévenir ou combattre les irritations de la peau ou des muqueuses, les pellicules, les érythèmes ou les prurits ;
- dans le traitement des troubles neurologiques de la peau tels que lichens, prurigos, toxidermies prurigineuses, prurits sévères d'origine neurogène ;
- dans le traitement des ulcères et de toutes maladies causées par Helicobacter Pylori ou une bactérie uréase positive gram négative ;
- dans le traitement des maladies causées par l'angiogénèse ou dont l'angiogénèse est un symtôme ;
- dans le traitement des algies oculaires et/ou palbébrales et/ou les dysesthésies oculaires ou palpébrales ;
- comme antiperspirant.

La présente invention inclut aussi une méthode pour traiter lesdites affections aux doses indiquées ci-dessus.

Les compositions pharmaceutiques selon la présente invention peuvent également renfermer d'autres produits actifs utiles pour traiter les maladies ou troubles indiqués ci-dessus, par exemple, des bronchodilatateurs, des antitussifs, des antihistaminiques, des antiinflammatoires, des antiémétiques, des agents de chimiothérapie.

Les Préparations et Exemples suivants illustrent l'invention sans toutefois la limiter.

Dans les Préparations et dans les Exemples on utilise les abréviations suivantes :

DMF : diméthylformamide
DMSO : diméthylsulfoxyde
DCM : dichlorométhane
THF : tétrahydrofurane
éther chlorhydrique : solution saturée d'acide chlorhydrique dans l'éther
BOP : benzotriazol-1-yloxytris(diméthylamino)phosphonium hexafluorophosphate
F : point de fusion
TA : température ambiante
Teb : température d'ébullition
silice H : gel de silice 60H commercialisé par Merck (DARMSTAD)

Les spectres de résonance magnétique nucléaire du proton (RMN $^1$H) sont enregistrés à 200 MHz dans DMSO-$d_6$, en utilisant le pic du DMSO-$d_6$ comme référence. Les déplacements chimiques δ sont indiqués en parties par million (ppm). Les signaux observés sont exprimés ainsi :

s : singulet ; se : singulet élargi ; t : triplet ; qd : quadruplet ; m : massif : mt : multiplet.

Les spectres RMN confirment les structures des composés.

PREPARATIONS

1. Préparation des composés de formule (VI).

Préparation 1.1

Acide 2-(2,3-dichlorophényl)acétique.

(VI) : B = $-CH_2-$ ;

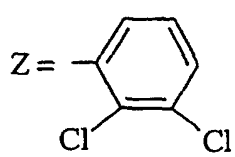

A) Ester méthylique de l'acide 2,3-dichlorobenzoïque.

A une solution de 25,08 g d'acide 2,3-dichlorobenzoïque dans 125 ml de MeOH on ajoute 6 ml d'acide sulfurique concentré puis chauffe à reflux pendant une nuit. On concentre sous vide le mélange réactionnel, reprend le résidu à l'eau, alcalinise par ajout d'une solution à 10 % de $NaHCO_3$, extrait à l'éther, lave deux fois la phase organique à l'eau, sèche sur $Na_2SO_4$ et évapore le solvant sous vide. On obtient 25,68 g du produit attendu.

B) Alcool 2,3-dichlorobenzylique.

On refroidit à 0°C une suspension de 10,56 g d'hydrure d'aluminium et de lithium dans 125 ml de THF, ajoute, goutte à goutte, une solution de 25,68 g du composé obtenu à l'étape précédente dans 100 ml de THF, laisse remonter la température à TA et laisse 2 heures sous agitation à TA. On dilue le mélange réactionnel par ajout de 250 ml de THF et hydrolyse par ajout de 11 ml d'eau, 11 ml de NaOH 4N et 33 ml d'eau. On abandonne une nuit à TA, filtre les sels minéraux et concentre sous vide le filtrat. On obtient 21,54 g du produit attendu après séchage sous vide à 30°C.

C) Méthanesulfonate de 2,3-dichlorobenzyle.

On refroidit au bain de glace une solution de 21,54 g du composé obtenu à l'étape précédente et 18,6 ml de triéthylamine dans 150 ml de DCM, ajoute, goutte à goutte, une solution de 10,4 ml de chlorure de méthanesulfonyle dans 50 ml de DCM à une température inférieure à 10°C et laisse sous agitation en laissant remonter la température à TA. On concentre sous vide, extrait le résidu à l'éther, lave deux fois par une solution tampon pH = 2, par une solution saturée de NaCl, sèche sur $Na_2SO_4$ et évapore le solvant sous vide. On obtient 29,25 g du produit attendu.

D) 2,3-Dichlorophénylacétonitrile.

A une solution de 29,25 g du composé obtenu à l'étape précédente dans 200 ml d'EtOH et 50 ml d'eau, on ajoute 10,1 g de cyanure de potassium à 97 % puis chauffe à reflux pendant 2 heures. On concentre sous vide, extrait le résidu à l'AcOEt, lave quatre fois la phase organique à l'eau, par une solution saturée de NaCl, sèche sur $Na_2SO_4$ et évapore le solvant sous vide. On reprend le résidu dans 200 ml de pentane et laisse une nuit en cristallisation sous agitation. On essore le précipité formé et le sèche sous vide. On obtient 17,17 g du produit attendu.

E) Acide 2-(2,3-dichlorophényl)acétique.

A une solution de 17,17 g du composé obtenu à l'étape précédente dans 188 ml d'EtOH on ajoute une solution de 24,23 g de KOH dans 74 ml d'eau, puis chauffe une nuit à reflux. On concentre sous vide, reprend le résidu dans 100 ml d'eau, lave trois fois la phase aqueuse à l'éther, acidifie la phase aqueuse à pH = 1 par ajout d'une solution concentré d'HCl et laisse en cristallisation sous agitation en refroidissant au bain de glace. On essore le précipité formé, le lave à l'eau et le sèche sous vide à 40°C. On obtient 17,17 g du produit attendu.

2. Préparations des composés de formule (II).

Préparation 2.1

2-[4-Benzoyl-2-(3,4-dichlorophényl)morpholin-2-yl]acétaldéhyde, isomère unique.

(II) : B = liaison directe ;

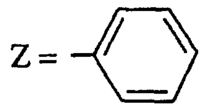

A) Benzoate de 2-[2-(3,4-dichlorophényl)morpholin-2-yl)éthyle, isomère lévogyre.

On prépare ce composé selon le mode opératoire décrit à la Préparation 1.1 dans WO 00/58292.

B) [2-(3,4-dichlorophényl)-2-(2-hydroxyéthyl)morpholin-4-yl](phényl)méthanone, isomère unique.

On refroidit à 0°C une solution de 4 g du composé obtenu à l'étape précédente et 1,5 ml de triéthylamine dans 100 ml de DCM, ajoute, goutte à goutte, une solution de 1,41 g de chlorure de benzoyle dans 10 ml de DCM et laisse 30 minutes sous agitation. On concentre sous vide le mélange réactionnel, extrait le résidu à l'éther, lave la phase organique à l'eau, par une solution tampon pH = 2, à l'eau, par une solution saturée de NaCl, sèche sur $Na_2SO_4$ et évapore le solvant sous vide. On reprend le résidu huileux ainsi obtenu dans 70 ml d'EtOH 95, ajoute 2,5 ml d'une solution à 30 % de NaOH et laisse 1 heure sous agitation à TA. On concentre sous vide, extrait le résidu à l'AcOEt, lave trois fois la phase organique à l'eau, par une solution saturée de NaCl, sèche sur $Na_2SO_4$ et évapore le solvant sous vide. On obtient 4 g du produit attendu.

C) 2-[4-Benzoyl-2-(3,4-dichlorophényl)morpholin-2-yl]acétaldéhyde, isomère unique.

On refroidit à -60°C, sous atmosphère d'azote, une solution de 1,85 g du composé obtenu à l'étape précédente et 2,25 ml de DMSO dans 25 ml de DCM, ajoute goutte à goutte 1,38 ml de chlorure d'oxalyle et laisse 2 heures sous agitation à -60°C. On ajoute ensuite 4,42 ml de triéthylamine et laisse sous agitation en laissant remonter la température à TA. On dilue le mélange réactionnel par ajout de DCM, lave la phase organique à l'eau, par une solution à 10 % de $Na_2CO_3$, deux fois à l'eau, par une solution saturée de NaCl, sèche sur $Na_2SO_4$ et évapore le solvant sous vide. On obtient 1,7 g du produit attendu.

Préparation 2.2

2-[4-(2,3-dichlorobenzoyl)-2-(3,4-dichlorophényl)morpholin-2-yl]acétaldéhyde, isomère unique.

(II) : B = liaison directe ;

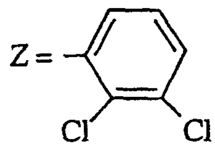

A) (2,3-Dichlorophényl)[2-(3,4-dichlorophényl)-2-(2-hydroxyéthyl)morpholin-4-yl]méthanone, isomère unique.

A une solution de 2,5 g du composé obtenu à l'étape A) de la Préparation 2.1, 1,2 g d'acide 2,3-dichlorobenzoïque et 0,75 g de triéthylamine dans 50 ml de DCM, on ajoute 3,3 g de BOP et laisse 30 minutes sous agitation à TA. On concentre sous vide, extrait le résidu à l'AcOEt, lave la phase organique à l'eau, par une solution tampon pH = 2, à l'eau, sèche sur $Na_2SO_4$ et évapore le solvant sous vide. On reprend le résidu dans 30 ml de MeOH, ajoute 3 ml d'une solution à 30 % de NaOH, et laisse 30 minutes sous agitation à TA. On concentre sous vide, extrait le résidu à l'éther, lave la phase organique à l'eau, sèche sur $Na_2SO_4$ et évapore le solvant sous vide. On chromatographie le résidu sur gel de silice H en éluant par le gradient du mélange DCM/MeOH de (100/0,1 ; v/v) à (100/1 ; v/v). On obtient 1,55 g du produit attendu.

B) 2-[4-(2,3-Dichlorobenzoyl)-2-(3,4-dichlorophényl)morpholin-2-yl]acétaldéhyde, isomère unique.

On refroidit à -60°C une solution de 1,5 g du composé obtenu à l'étape précédente et 1,5 g de DMSO dans 20 ml de DCM, ajoute goutte à goutte 1,25 g de chlorure d'oxalyle et laisse 1 heure sous agitation à -60°C. On ajoute ensuite 2 g de triéthylamine et laisse sous agitation en laissant remonter la température à TA. On extrait le mélange réactionnel au DCM, lave la phase organique par une solution d'HCl 1N, à l'eau, sèche sur $Na_2SO_4$ et évapore le solvant sous vide. On obtient 1,4 g du produit attendu.

Préparation 2.3

2-[2-(3,4-Dichlorophényl)-4-[2-(2,6-dichlorophényl)acétyl]morpholin-2-yl]acétaldéhyde, isomère unique.

(II) : B = $-CH_2-$ ;

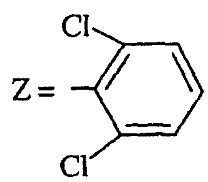

A) Benzoate de 2-[2-(3,4-dichlorophényl)-4-[2-(2,6-dichlorophényl)acétyl]morpholin-2-yl]éthyle, isomère unique.

On refroidit à 0°C une solution de 4 g du composé obtenu à l'étape A de la Préparation 2.1 dans 43 ml de DCM, ajoute 2,16 g d'acide 2-(2,6-dichlorophényl) acétique, puis une solution de 3 ml de triéthylamine dans 50 ml de DCM et 4,7 g de BOP puis laisse sous agitation en laissant remonter la température à TA. On concentre sous vide, extrait le résidu à l'AcOEt, lave la phase organique par une solution d'HCl 2N, à l'eau, par une solution à 10 % de $Na_2CO_3$, à l'eau, par une solution saturée de NaCl, sèche sur $Na_2SO_4$ et évapore le solvant sous vide. On obtient 6 g du produit attendu.

B) 2-(2,6-Dichlorophényl)-1-[2-(3,4-dichlorophényl)-2-(2-hydroxyéthyl)morpholin-4-yl]-1-éthanone, isomère unique.

On chauffe à reflux un mélange de 6 g du composé obtenu à l'étape précédente dans 100 ml de MeOH, ajoute 3,5 ml d'une solution à 30 % de NaOH et laisse 1 heure à reflux sous agitation. On concentre sous vide, reprend le résidu à l'eau, extrait à l'AcOEt, lave deux fois la phase organique à l'eau, par une solution saturée de NaCl, sèche sur $Na_2SO_4$ et évapore le solvant sous vide. On chromatographie le résidu sur gel de silice H en éluant au DCM puis par le gradient du mélange DCM/MeOH de (100/1 ; v/v) à (100/3 ; v/v). On obtient 2,42 g du produit attendu.

C) 2-[2-(3,4-Dichlorophényl)-4-[2-(2,6-dichlorophényl)acétyl]morpholin-2-yl]acétaldéhyde, isomère unique.

On refroidit à -60°C un mélange de 0,6 ml de chlorure d'oxalyle dans 11 ml de DCM, ajoute une solution de 1,2 ml de DMSO dans 5 ml de DCM, puis, goutte à goutte, une solution de 2,42 g du composé obtenu à l'étape

précédente et 1,6 ml de DMSO dans 11 ml de DCM et laisse 30 minutes sous agitation à -50°C. On ajoute ensuite 4,6 ml de triéthylamine et laisse sous agitation en laissant remonter la température à TA. On extrait le mélange réactionnel au DCM, lave la phase organique par une solution d'HCl 2N, à l'eau, par une solution à 10 % de $Na_2CO_3$, à l'eau, par une solution saturée de NaCl, sèche sur $Na_2SO_4$ et évapore sous vide le solvant. On obtient 2,24 g du produit attendu.

Préparation 2.4

2-[2-(3,4-Dichlorophényl)-4-[2-(2,3-dichlorophényl)acétyl]morpholin-2-yl] acétaldéhyde, isomère unique.

(II) : B = $-CH_2-$ ; Z = 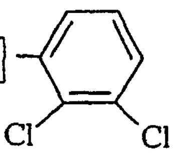

A) Benzoate de 2-[2-(3,4-dichlorophényl)-4-[2-(2,3-dichlorophényl)acétyl]morpholin-2-yl]éthyle, isomère unique.

On prépare ce composé selon le mode opératoire décrit à l'étape A de la Préparation 2.3 à partir de 4,9 g du composé obtenu à l'étape A de la Préparation 2.1 dans 52 ml de DCM, de 2,67 g du composé obtenu à la Préparation 1.1, d'une solution de 3,62 ml de triéthylamine dans 36 ml de DCM et de 5,76 g de BOP. On obtient 7,11 g du produit attendu.

B) 2-[2,3-Dichlorophényl)-1-[2-(3,4-dichlorophényl)-2-(2-hydroxyéthyl)morpholin-4-yl]-1-éthanone, isomère unique.

A une solution de 7,11 g du composé obtenu à l'étape précédente dans 100 ml de MeOH on ajoute 5 ml d'une solution à 30 % de NaOH et laisse 1 heure sous agitation à TA. On concentre sous vide, extrait le résidu à l'AcOEt, lave deux fois la phase organique à l'eau, par une solution saturée de NaCl, sèche sur $Na_2SO_4$ et évapore le solvant sous vide. On chromatographie le résidu sur gel de silice H en éluant au DCM puis par le mélange DCM/MeOH (100/1 ; v/v). On obtient 2,21 g du produit attendu.

C) 2-[2-(3,4-Dichlorophényl)-4-[2-(2,3-dichlorophényl)acétyl]morpholin-2-yl] acétaldéhyde, isomère unique.

On prépare ce composé selon le mode opératoire décrit à l'étape C de la Préparation 2.3 à partir de 0,5 ml de chlorure d'oxalyle dans 10 ml de DCM, d'une solution de 1,02 ml de DMSO dans 5 ml de DCM, d'une solution de 2,21 g du composé obtenu à l'étape précédente et 1,43 ml de DMSO dans 10 ml de DCM et de 4,2 ml de triéthylamine. On obtient 2,1 g du produit attendu.

3. Préparations des composés de formule (III).

Préparation 3.1

N,N-Diméthyl-4-(pipéridin-1-yl)pipéridine-4-carboxamide.

$$(III) : R_1 = -CH_3.$$

A) 1-Benzyl-4-cyano-4-(pipéridin-1-yl)pipéridine.

A une solution de 18,6 g de 1-benzylpipéridin-4-one et 12,16 g de chlorhydrate de pipéridine dans 25 ml de MeOH et 25 ml d'eau, on ajoute, goutte à goutte et à TA, une solution de 5,3 g de cyanure de sodium dans 20 ml d'eau et laisse 48 heures sous agitation à TA. On essore le précipité formé, le lave à l'eau et le sèche sous vide. On obtient 27 g du produit attendu.

B) 1-Benzyl-4-(pipéridin-1-yl)pipéridine-4-carboxamide.

On ajoute 28,3 g du composé obtenu à l'étape précédente dans 80 ml d'acide sulfurique à 95 % et chauffe à 100°C pendant 10 minutes. Après refroidissement à TA, on verse le mélange réactionnel sur de la glace, amène à pH = 7 par ajout d'une solution à 25 % de $NH_4OH$, extrait au DCM, lave la phase organique à l'eau, par une solution saturée de NaCl, sèche sur $Na_2SO_4$ et évapore le solvant sous vide. On reprend le résidu dans l'acétone, laisse 2 heures sous agitation à TA et essore le précipité formé. On obtient 20,8 g du produit attendu.

C) N,N-Diméthyl-1-benzyl-4-(pipéridin-1-yl)pipéridine-4-carboxamide et N-méthyl-1-benzyl-4-(pipéridin-1-yl)pipéridine-4-carboxamide.

A une suspension de 3,6 g d'hydrure de sodium à 60 % dans l'huile dans 120 ml de THF, on ajoute, goutte à goutte et à TA, une solution de 9,87 g du composé obtenu à l'étape précédente dans 120 ml de THF et chauffe à 60°C pendant 2 heures. Après refroidissement à TA, on ajoute, goutte à goutte, une solution de 8,52 g d'iodure de méthyle dans 60 ml de DMF et laisse 4 heures sous agitation à TA. On verse le mélange réactionnel sur de la glace, extrait à l'éther, lave la phase organique à l'eau, sèche sur $Na_2SO_4$ et évapore le solvant sous vide. On chromatographie le résidu sur gel de silice H en éluant par le mélange DCM/MeOH/$NH_4OH$ (100/1/0,1 ; v/v/v) et sépare :

- le composé le moins polaire : on obtient 6 g du N,N-diméthyl-1-benzyl-4-(pipéridin-1-yl)pipéridine-4-carboxamide ;
- le composé le plus polaire : on obtient 2,6 g du N-méthyl-1-benzyl-4-(pipéridine-1-yl)pipéridine-4-carboxamide.

D) N,N-Diméthyl-4-(pipéridin-1-yl)pipéridine-4-carboxamide.

On laisse 3 heures sous agitation à TA un mélange de 5,9 g du composé le moins polaire obtenu à l'étape précédente, 3,4 g de formiate d'ammonium et 1,5 g de palladium sur charbon à 10 % dans 60 ml de MeOH. On filtre le catalyseur sur Célite® et concentre sous vide le filtrat. On obtient 1,9 g du produit attendu après séchage sous vide à 60°C.

Préparation 3.2

Formiate de N-méthyl-4-(pipéridin-1-yl)pipéridine-4-carboxamide.

$$(III), HCOOH : R_1 = H.$$

On laisse 30 minutes sous agitation à TA un mélange de 4 g du composé le plus polaire obtenu à l'étape C de la Préparation 3.1, 2,43 g de formiate d'ammonium et 1 g de palladium sur charbon à 10 % dans 50 ml de MeOH. On filtre le catalyseur sur Célite® et concentre le filtrat sous vide. On obtient 2,6 g du produit attendu après séchage sous vide.

EXEMPLE 1

Dichlorhydrate de N,N-diméthyl-1-[2-[4-benzoyl-2-(3,4-dichlorophényl)morpholin-2-yl]éthyl]-4-(pipéridin-1-yl)pipéridine-4-carboxamide, isomère dextrogyre.

$$(I), 2HCl: R_1 = -CH_3 \text{ ; B = liaison directe ;}$$

Z =
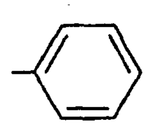

A une solution de 0,8 g du composé obtenu à la Préparation 2.1 dans 15 ml de DCM, on ajoute 0,6 g du composé obtenu à la Préparation 3.1 puis 0,9 g de triacétoxyborohydrure de sodium et 8 gouttes d'acide acétique et laisse une nuit sous agitation à TA. On alcalinise le mélange réactionnel par ajout d'une solution à 10 % de $Na_2CO_3$, extrait au DCM, lave trois fois la phase organique à l'eau, par une solution saturée de NaCl, sèche sur $Na_2SO_4$ et évapore le solvant sous vide. On chromatographie le résidu sur gel de silice H en éluant par le gradient du mélange DCM/MeOH de (100/0,5 ; v/v) à (100/2 ; v/v). On reprend le produit obtenu dans de l'éther chlorhydrique et évapore le solvant sous vide. On obtient 0,45 g du produit attendu après cristallisation dans le mélange pentane/éther iso. $\alpha_D^{20}$ = + 14,4° (c = 0,25 ; MeOH).

RMN $^{1}H$ : DMSO-$d_6$ + TFA, 350°K : δ (ppm) : 1,3 à 1,8 : m : 6H ; 2,0 à 3,3 : m : 20H ; 3,3 à 4,2 : m : 8H ; 7,2 à 7,7 : m : 8H.

EXEMPLE 2

Dichlorhydrate de N-méthyl-1-[2-[4-benzoyl-2-(3,4-dichlorophényl)morpholin-2-yl]éthyl]-4-(pipéridin-1-yl)pipéridine-4-carboxamide, isomère dextrogyre.

(I), 2HCl : $R_1$ = H ; B = liaison directe ; Z = 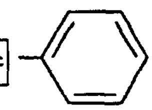

On prépare le composé selon le mode opératoire décrit à l'Exemple 1 à partir de 0,58 g du composé obtenu à la Préparation 2.1, 15 ml de DCM, 0,345 g du composé obtenu à la Préparation 3.2, 0,65 g de triacétoxyborohydrure de sodium et 8 gouttes d'acide acétique. On obtient 0,6 g du produit attendu après cristallisation dans le mélange pentane/éther iso.
$\alpha_D^{20}$ = + 13,6° (c = 0,25 ; MeOH).

EXEMPLE 3

Dichlorhydrate de N,N-diméthyl-1-[2-[4-(2,3-dichlorobenzoyl)-2-(3,4-dichlorophényl)morpholin-2-yl]éthyl]-4-(pipéridin-1-yl)pipéridine-4-carboxamide, isomère lévogyre.

(I), 2HCl : $R_1$ = $-CH_3$ ; B = liaison directe ; Z = 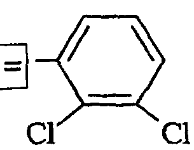

On prépare ce composé selon le mode opératoire décrit à l'Exemple 1 à partir de 0,75 g du composé obtenu à la Préparation 2.2, 20 ml de DCM, 0,43 g du composé obtenu à la Préparation 3.1, 0,7 g de triacétoxyborohydrure de sodium et 8 gouttes d'acide acétique. On obtient 0,8 g du produit attendu après cristallisation dans le mélange DCM/éther.
$\alpha_D^{20}$ = - 5,4° (c = 0,5 ; MeOH).

EXEMPLE 4

Dichlorhydrate de N,N-diméthyl-1-[2-[4-[(2,6-dichlorophényl)acétyl]-2-(3,4-dichlorophényl)morpholin-2-yl]éthyl]-4-(pipéridin-1-yl)pipéridine-4-carboxamide, isomère dextrogyre.

(I), 2HCl : $R_1$ = $-CH_3$ ; B = $-CH_2-$ ; Z = 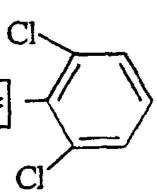

On prépare ce composé selon le mode opératoire décrit à l'Exemple 1 à partir de 0,45 g du composé obtenu

à la Préparation 2.3, 50 ml de DCM, 0,28 g du composé obtenu à la Préparation 3.1, 0,424 g de triacétoxyborohydrure de sodium et 3 gouttes d'acide acétique. On obtient 0,419 g du produit attendu après cristallisation dans l'éther. $\alpha_D^{20}$ = + 7,6° (c = 0,25 ; MeOH).

EXEMPLE 5 Dichlorhydrate de N,N-diméthyl-1-[2-[4-[2-(2,3-dichlorophényl)acétyl]-2-(3,4-dichlorophényl)morpholin-2-yl]éthyl]-4-(pipéridin-1-yl)pipéridine-4-carboxamide, isomère dextrogyre, dihydrate.

(I), 2HCl : $R_1$ = $-CH_3$ ; B = $-CH_2-$ ; Z =

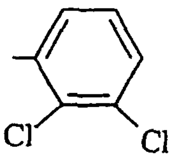

On prépare ce composé selon le mode opératoire décrit à l'Exemple 1 à partir de 0,5 g du composé obtenu à la Préparation 2.4, 7 ml de DCM, 0,312 g du composé obtenu à la Préparation 3.1, 0,47 g de triacétoxyborohydrure de sodium et 3 gouttes d'acide acétique. On obtient 0,446 g du produit attendu après cristallisation dans l'éther. $\alpha_D^{20}$ = +8,8° (c = 0,25 ; MeOH).

EXEMPLE 6

Dichlorhydrate de N-méthyl-1-[2-[4-[2-(2,3-dichlorophényl)acétyl]-2-(3,4-dichlorophényl)morpholin-2-yl]éthyl]-4-(pipéridin-1-yl)pipéridine-4-carboxamide, isomère dextrogyre, dihydrate.

(I), 2HCl : $R_1$ = H ; B = $-CH_2-$ ; Z =

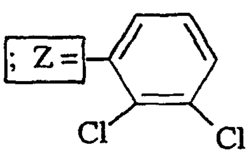

On prépare ce composé selon le mode opératoire décrit à l'Exemple 1 à partir de 0,6 g du composé obtenu à la Préparation 2.4, 60 ml de DCM, 0,3 g du composé obtenu à la Préparation 3.2, 0,56 g de triacétoxyborohydrure de sodium et 3 gouttes d'acide acétique. On obtient 0,556 g du produit attendu après cristallisation dans l'éther. $\alpha_D^{20}$ = + 8° (c = 0,25 ; MeOH).

## Revendications

**1.** Composé de formule:

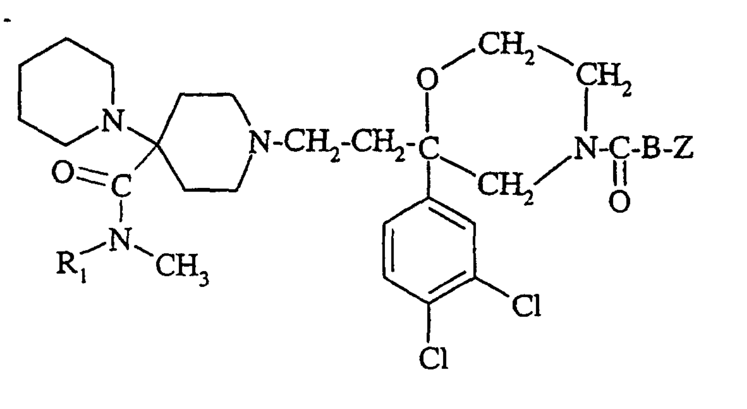

(I)

dans laquelle :

- $R_1$ représente un atome d'hydrogène ou un radical méthyle ;
- B représente une liaison directe ou un groupe $-CH_2-$ ;
- Z représente un phényle, un 2,3-dichlorophényle ou un 2,6-dichlorophényle ;

et ses sels avec des acides minéraux ou organiques, ses solvats et/ou ses hydrates.

**2.** Composé de formule (I) selon la revendication 1 sous forme d'isomères optiquement purs.

**3.** Un composé selon la revendication 1 ou 2 choisi parmi :

- N,N-diméthyl-1-[2-[4-benzoyl-2-(3,4-dichlorophényl) morpholin-2-yl]éthyl]-4-(pipéridin-1-yl)pipéridine-4-carboxamide, isomère dextrogyre ;
- N-méthyl-1-[2-[4-benzoyl-2-(3,4-dichlorophényl)morpholin-2-yl]éthyl]-4-(pipéridin-1-yl)pipéridine-4-carboxamide, isomère dextrogyre ;
- N,N-diméthyl-1-[2-[4-(2,3-dichlorobenzoyl)-2-(3,4-dichlorophényl)morpholin-2-yl]éthyl]-4-(pipéridin-1-yl)pipéridine-4-carboxamide, isomère lévogyre ;
- N,N-diméthyl-1-[2-[4-(2,6-dichlorophényl)acétyl]-2-(3,4-dichlorophényl)morpholin-2-yl]éthyl]-4-(pipéridin-1-yl)pipéridine-4-carboxamide, isomère dextrogyre ;
- N,N-diméthyl-1-[2-[4-[2-(2,3-dichlorophényl)acétyl]-2-(3,4-dichlorophényl)morpholin-2-yl]éthyl]-4-(pipéridin-1-yl)pipéridine-4-carboxamide, isomère dextrogyre ;
- N-méthyl-1-[2-[4-[2-(2,3-dichlorophényl)acétyl]-2-(3,4-dichlorophényl)morpholin-2-yl]éthyl]-4-(pipéridin-1-yl) pipéridine-4-carboxamide, isomère dextrogyre ; et ses sels avec des acides minéraux ou organiques, ses solvats et/ou ses hydrates.

**4.** Composé selon l'une quelconque des revendications 1 à 3 qui est le :

- N,N-diméthyl-1-[2-[4-benzoyl-2-(3,4-dichlorophényl) morpholin-2-yl]éthyl]-4-(pipéridin-1-yl)pipéridine-4-carboxamide, isomère dextrogyre ; et ses sels avec des acides minéraux ou organiques, ses solvats et/ou ses hydrates.

**5.** Procédé pour la préparation des composés de formule (I) selon la revendication 1, de leurs sels, leurs solvats et/ou leurs hydrates, **caractérisé en ce que** :

on fait réagir un composé de formule :

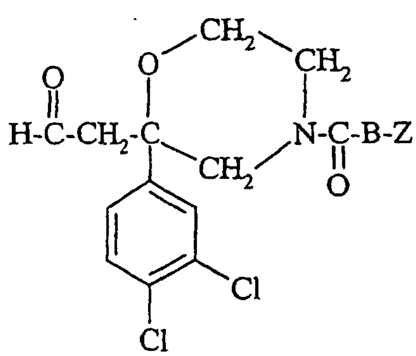

(II)

dans laquelle B et Z sont tels que définis pour un composé de formule (I) selon la revendication 1, avec un composé de formule :

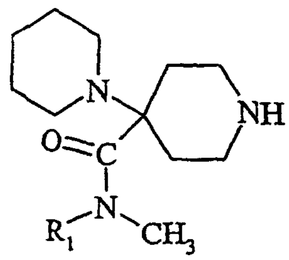

(III)

dans laquelle $R_1$ est tel que défini pour un composé de formule (I) selon la revendication 1, en présence d'un acide, dans un solvant, puis on réduit le sel d'iminium formé intermédiairement au moyen d'un agent réducteur.

6. Procédé pour la préparation des composés de formule (I) selon la revendication 1, de leurs sels, leur solvats et/ou leurs hydrates, **caractérisé en ce que** :

on fait réagir un composé de formule :

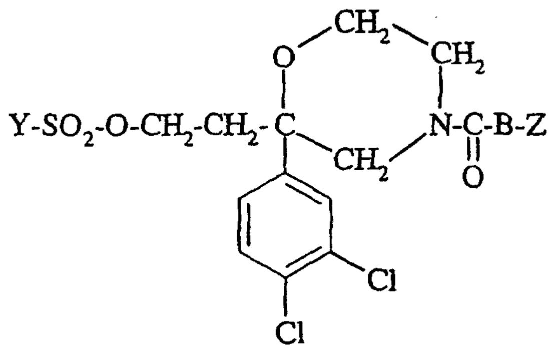

(IV)

dans laquelle B et Z sont tels que définis pour un composé de formule (I) dans la revendication 1 et Y représente un groupe méthyle, phényle, tolyle ou trifluorométhyle, avec un composé de formule :

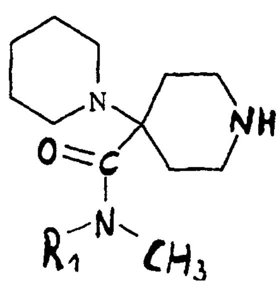

(III)

dans laquelle $R_1$ est tel que défini pour un composé de formule (I) dans la revendication 1.

7. Procédé pour la préparation des composés de formule (I) selon la revendication 1, de leurs sels, leur solvats et/ou leurs hydrates, **caractérisé en ce que** :

on fait réagir un composé de formule :

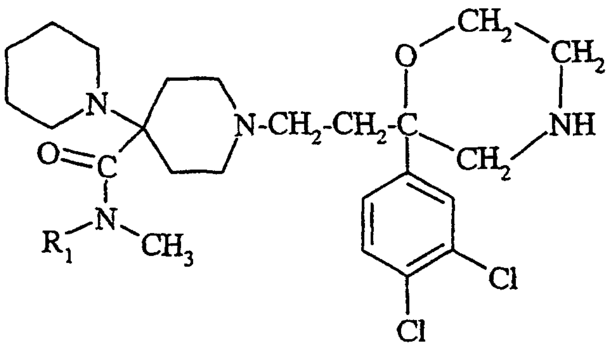

(V)

dans laquelle $R_1$ et tel que défini pour un composé de formule (I) selon la revendication1, avec un dérivé fonctionnel d'un acide de formule :

HOOC-B-Z (VI)

dans laquelle B et Z sont tels que définis pour un composé de formule (I) selon la revendication 1.

**8.** Composition pharmaceutique comprenant en tant que principe actif un composé selon l'une quelconque des revendications 1 à 4 ou l'un de ses sels, solvats et/ou hydrates pharmaceutiquement acceptables.

**9.** Composition pharmaceutique selon la revendication 8, contenant de 0,1 à 1000 mg de principe actif, sous forme d'unité de dosage dans laquelle le principe actif est mélangé à au moins un excipient pharmaceutique.

**10.** Utilisation d'un composé selon l'une quelconque des revendications 1 à 4 ou l'un de ses sels, solvats et/ou hydrates pharmaceutiquement acceptables pour la préparation de médicaments destinés à traiter toute pathologie où soit la neurokinine A et/ou les récepteurs $NK_2$, soit la neurokinine B et/ou les récepteurs $NK_3$, soit à la fois la neurokinine A et la neurokinine B et/ou les récepteurs $NK_2$ et $NK_3$ sont impliqués.

**11.** Utilisation selon la revendication 10, pour la préparation de médicaments destinés à traiter les pathologies du système respiratoire, gastro-intestinal, urinaire, immunitaire, cardiovasculaire, du système nerveux central ainsi que la douleur, la migraine, les inflammations, les nausées et les vomissements, les maladies de la peau.

**12.** Utilisation selon la revendication 11 pour la préparation de médicaments destinés à traiter la bronchite chronique obstructive, l'asthme, l'incontinence urinaire, le syndrome du colon irritable, la maladie de Crohn, les colites ulcératives, la dépression, l'anxiété, l'épilepsie, la schizophrénie.

**13.** Médicament **caractérisé en ce qu'**il comprend un composé selon l'une quelconque des revendications 1 à 4 ou l'un de ses sels, solvats et/ou hydrates pharmaceutiquement acceptables.

## Patentansprüche

**1.** Verbindung der Formel:

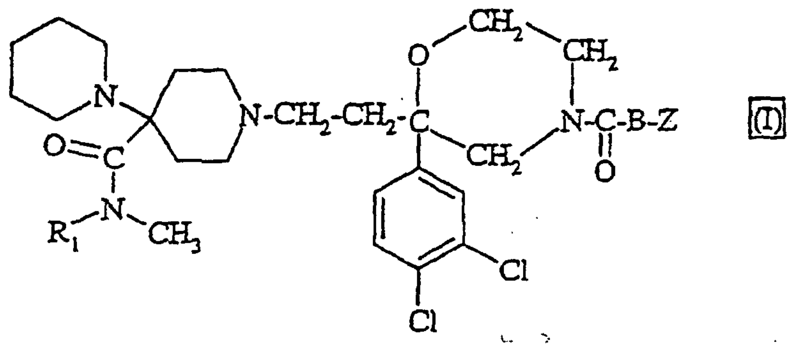

in der:

- $R_1$ ein Wasserstoffatom oder eine Methylgruppe bedeutet;
- B eine direkte Bindung oder eine Gruppe $-CH_2-$ darstellt;
- Z Phenyl, 2,3-Dichlorphenyl oder 2,6-Dichlorphenyl bedeutet;

und deren Salze mit anorganischen oder organischen Säuren, deren Solvate und/oder deren Hydrate.

**2.** Verbindung der Formel (I) nach Anspruch 1 in Form der optisch reinen Isomeren.

**3.** Verbindung nach Anspruch 1 oder 2, ausgewählt aus:

- N,N-Dimethyl-1-[2-[4-benzoyl-2-(3,4-dichlorphenyl)-morpholin-2-yl]-ethyl]-4-(piperidin-1-yl)-piperidin-4-carboxamid, rechtsdrehendes Isomeres;
- N-Methyl-1-[2-[4-benzoyl-2-(3,4-dichlorphenyl)-morpholin-2-yl]-ethyl]-4-(piperidin-1-yl)-piperidin-4-carboxamid, rechtsdrehendes Isomeres;
- N,N-Dimethyl-1-[2-[4-(2,3-dichlorbenzoyl)-2-(3,4-dichlorphenyl)-morpholin-2-yl]-ethyl]-4-(piperidin-1-yl)-piperidin-4-carboxamid, linksdrehendes Isomeres;
- N,N-Dimethyl-1-[2-[4-(2,6-dichlorphenyl)-acetyl]-2-(3,4-dichlorphenyl)-morpholin-2-yl]-ethyl]-4-(piperidin-1-yl)-piperidin-4-carboxamid, rechtsdrehendes Isomeres;
- N,N-Dimethyl-1-[2-[4-[2-(2,3-dichlorphenyl)-acetyl]-2-(3,4-dichlorphenyl)-morpholin-2-yl]-ethyl]-4-(piperidin-1-yl)-piperidin-4-carboxamid, rechtsdrehendes Isomeres;
- N-Methyl-1-[2-[4-[2-(2,3-dichlorphenyl)-acetyl]-2-(3,4-dichlorphenyl)-morpholin-2-yl]-ethyl]-4-(piperidin-1-yl)-piperidin-4-carboxamid, rechtsdrehendes Isomeres;

und deren Salze mit anorganischen oder organischen Säuren, deren Solvate und/oder deren Hydrate.

**4.** Verbindung nach einem der Ansprüche 1 bis 3, nämlich:

- N,N-Dimethyl-1-[2-[4-benzoyl-2-(3,4-dichlorphenyl)-morpholin-2-yl]-ethyl]-4-(piperidin-1-yl)-piperidin-4-carboxamid, rechtsdrehendes Isomeres;

und dessen Salze mit anorganischen oder organischen Säuren, dessen Solvate und/oder dessen Hydrate.

**5.** Verfahren zur Herstellung der Verbindungen der Formel (I) nach Anspruch 1, von deren Salzen, deren Solvaten und/oder deren Hydraten, **dadurch gekennzeichnet, daß** man eine Verbindung der Formel:

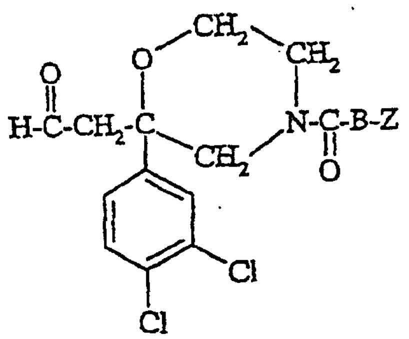

(II)

in der B und Z die bezüglich der Verbindung der Formel (I) nach Anspruch 1 angegebenen Bedeutungen besitzen, mit einer Verbindung der Formel:

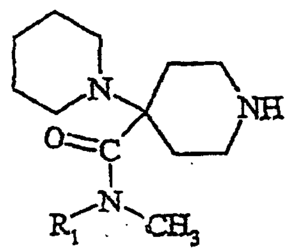

(III)

in der $R_1$ die bezüglich der Verbindung der Formel (I) in Anspruch 1 angegebenen Bedeutungen besitzt, in Gegenwart einer Säure und in einem Lösungsmittel umsetzt und dann das als Zwischenprodukt gebildete Iminiumsalz mit Hilfe eines Reduktionsmittels reduziert.

**6.** Verfahren zur Herstellung der Verbindungen der Formel (I) nach Anspruch 1, von deren Salzen, deren Solvaten und/deren Hydraten, **dadurch gekennzeichnet, daß** man eine Verbindung der Formel:

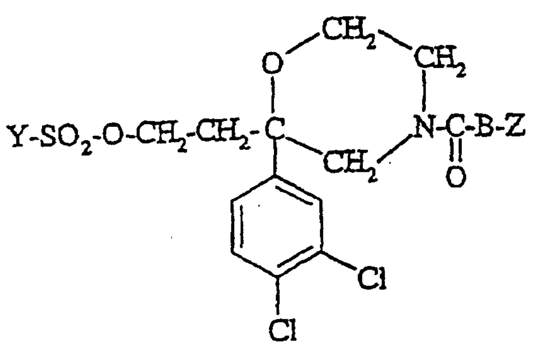

(IV)

in der B und Z die für die Verbindung der Formel (I) in Anspruch 1 angegebenen Bedeutungen besitzen und Y eine Methyl-, Phenyl-, Tolyl- oder TrifluormethylGruppe darstellt, mit einer Verbindung der Formel:

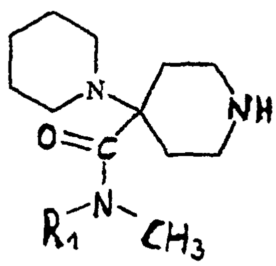

(III)

in der $R_1$ die für die Verbindung der Formel (I) nach Anspruch 1 angegebenen Bedeutungen besitzt, umsetzt.

**7.** Verfahren zur Herstellung der Verbindungen der Formel (I) nach Anspruch 1, von deren Salzen, deren Solvaten und/oder Hydraten, **dadurch gekennzeichnet, daß** man eine Verbindung der Formel:

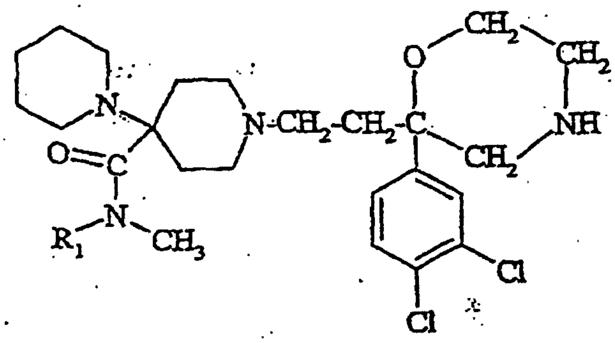

(V)

in der $R_1$ die bezüglich der Verbindung der Formel (I) in Anspruch 1 angegebenen Bedeutungen besitzt, mit einem funktionellen Derivat einer Säure der Formel:

$$HOOC\text{-}B\text{-}Z \qquad (VI)$$

in der B und Z die bezüglich der Verbindung der Formel (I) in Anspruch 1 angegebenen Bedeutungen besitzen, umsetzt.

**8.** Pharmazeutische Zubereitung umfassend als Wirkstoff eine Verbindung nach einem der Ansprüche 1 bis 4 oder eines ihrer pharmazeutisch annehmbaren Salze, Solvate und/oder Hydrate.

**9.** Pharmazeutische Zubereitung nach Anspruch 8, enthaltend 0,1 bis 1000 mg des Wirkstoffes in Form einer Dosiseinheit, in der der Wirkstoff in Mischung mit mindestens einem pharmazeutischen Hilfsstoff vorliegt.

**10.** Verwendung einer Verbindung nach einem der Ansprüche 1 bis 4 oder eines ihrer pharmazeutisch annehmbaren Salze, Solvate und/oder Hydrate für die Herstellung von Arzneimitteln zur Behandlung jeglicher pathologischer Zustände, bei denen Neurokinin A und/oder die Rezeptoren $NK_2$, oder Neurokinin B und/oder die Rezeptoren $NK_3$, oder gleichzeitig Neurokinin A und Neurokinin B und/oder die Rezeptoren $NK_2$ und $NK_3$ beteiligt sind.

**11.** Verwendung nach Anspruch 10 für die Herstellung von Arzneimitteln für die Behandlung von pathologischen Zuständen des Atmungssystems, des Magen-Darm-Systems, des Harnsystems, des Immunsystems, des kardiovaskulären Systems, des Zentralnervensystems sowie von Schmerzen, Migräne, Entzündungszuständen, Übelkeit und Erbrechen und Hautkrankheiten.

**12.** Verwendung nach Anspruch 11 für die Herstellung von Arzneimitteln zur Behandlung von chronischer obstruktiver Bronchitis, Asthma, Harninkontinenz, dem Reizdarm-Syndrom, der Crohnschen Krankheit, der ulcerativen Colitis, von Depressionen, der Angst, der Epilepsie und der Schizophrenie.

**13.** Arzneimittel, **dadurch gekennzeichnet, daß** es eine Verbindung nach einem der Ansprüche 1 bis 4 oder eines ihrer pharmazeutisch annehmbaren Salze, Solvate und/oder Hydrate umfaßt.

## Claims

**1.** Compound of formula:

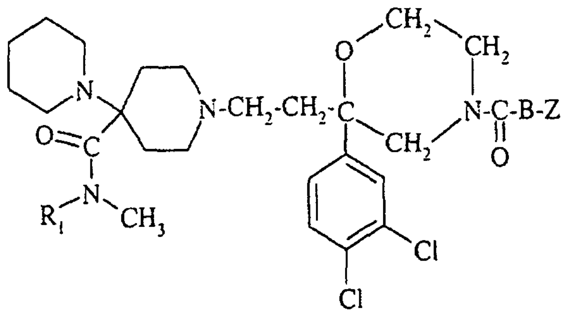

(I)

in which:

- $R_1$ represents a hydrogen atom or a methyl radical;
- B represents a direct bond or a $-CH_2-$ group;
- Z represents a phenyl, a 2,3-dichlorophenyl or a 2,6-dichlorophenyl;

and its salts with inorganic or organic acids, its solvates and/or its hydrates.

**2.** Compound of formula (I) according to Claim 1, in the form of optically pure isomers.

**3.** Compound according to Claim 1 or 2, chosen from:

- N,N-dimethyl-1-[2-[4-benzoyl-2-(3,4-dichlorophenyl)morpholin-2-yl]ethyl]-4-(piperidin-1-yl)piperidine-4-carboxamide, dextrorotatory isomer;
- N-methyl-1-[2-[4-benzoyl-2-(3,4-dichlorophenyl)-morpholin-2-yl]ethyl]-4-(piperidin-1-yl)piperidine-4-carboxamide, dextrorotatory isomer;
- N,N-dimethyl-1-[2-[4-(2,3-dichlorobenzoyl)-2-(3,4-dichlorophenyl)morpholin-2-yl]ethyl]-4-(piperidin-1-yl)piperidine-4-carboxamide, laevorotatory isomer;
- N,N-dimethyl-1-[2-[4-(2,6-dichlorophenyl)acetyl]-2-(3,4-dichlorophenyl)-morpholin-2-yl]ethyl]-4-(piperidin-1-yl)piperidine-4-carboxamide, dextrorotatory isomer;
- N,N-dimethyl-1-[2-[4-[2-(2,3-dichlorophenyl)-acetyl]-2-(3,4-dichlorophenyl)morpholin-2-yl]-ethyl]-4-(piperidin-1-yl)piperidine-4-carboxamide, dextrorotatory isomer;
- N-methyl-1-[2-[4-[2-(2,3-dichlorophenyl)acetyl]-2-(3,4-dichlorophenyl)morpholin-2-yl]ethyl]-4-(piperidin-1-yl) piperidine-4-carboxamide, dextrorotatory isomer;

and its salts with inorganic or organic acids, its solvates and/or its hydrates.

**4.** Compound according to any one of Claims 1 to 3, which is:

- N,N-dimethyl-1-[2-[4-benzoyl-2-(3,4-dichlorophenyl)morpholin-2-yl]ethyl]-4-(piperidin-1-yl)-piperidine-4-carboxamide, dextrorotatory isomer;

and its salts with inorganic or organic acids, its solvates and/or its hydrates.

**5.** Method for preparing the compounds of formula (I) according to Claim 1, their salts, their solvates and/or their hydrates, **characterized in that**:

a compound of formula:

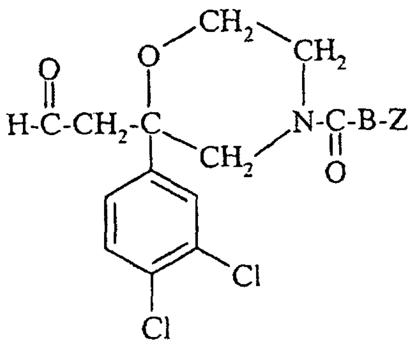

(II)

in which B and Z are as defined for a compound of formula (I) according to Claim 1, is reacted with a compound of formula:

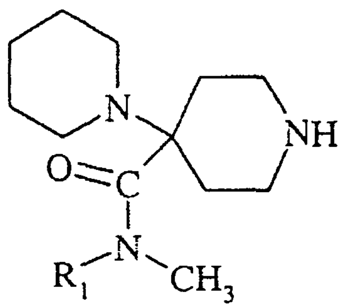

(III)

in which $R_1$ is as defined for a compound of formula (I) according to Claim 1, in the presence of an acid, in a solvent, and then the intermediate iminium salt formed is reduced by means of a reducing agent.

**6.** Method for preparing the compounds of formula (I) according to Claim 1, their salts, their solvates and/or their hydrates, **characterized in that**:

a compound of formula:

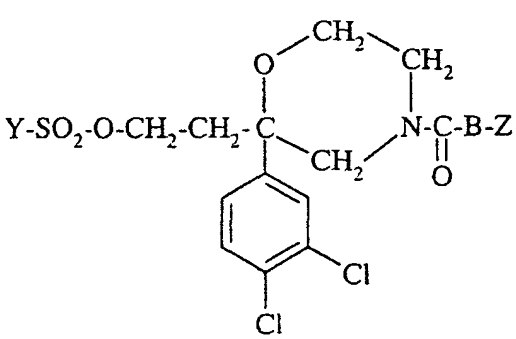

(IV)

in which B and Z are as defined for a compound of formula (I) in Claim 1 and Y represents a methyl, phenyl, tolyl or trifluoromethyl group, is reacted with a compound of formula:

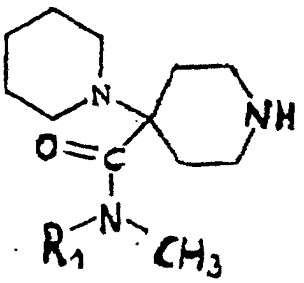

(III)

in which $R_1$ is as defined for a compound of formula (I) in Claim 1.

**7.** Method for preparing the compounds of formula (I) according to Claim 1, their salts, their solvates and/or their hydrates, **characterized in that**:

a compound of formula:

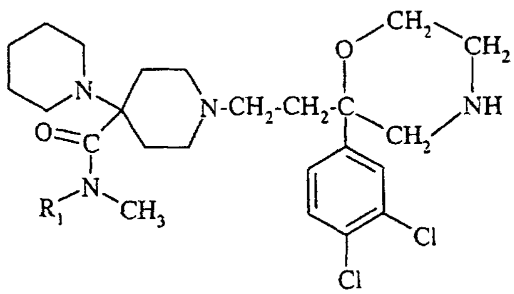

(V)

in which $R_1$ is as defined for a compound of formula (I) according to Claim 1, is reacted with a functional derivative of an acid of formula:

$$HOOC\text{-}B\text{-}Z \quad (VI)$$

in which B and Z are as defined for a compound of formula (I) according to Claim 1.

**8.** Pharmaceutical composition comprising, as active ingredient, a compound according to any one of Claims 1 to 4 or one of its pharmaceutically acceptable salts, solvates and/or hydrates.

**9.** Pharmaceutical composition according to Claim 8, containing from 0.1 to 1000 mg of active ingredient in dosage unit form in which the active ingredient is mixed with at least one pharmaceutical excipient.

**10.** Use of a compound according to any one of Claims 1 to 4 or one of its pharmaceutically acceptable salts, solvates and/or hydrates for the preparation of medicaments intended for treating any pathology where either neurokinin A and/or $NK_2$ receptors, or neurokinin B and/or $NK_3$ receptors, or both neurokinin A and neurokinin B and/or $NK_2$ and $NK_3$ receptors are involved.

**11.** Use according to Claim 10, for the preparation of medicaments intended for treating pathologies of the respiratory, gastrointestinal, urinary, immune and cardiovascular system and of the central nervous system as well as pain, migraine, inflammation, nausea and vomiting, and skin diseases.

**12.** Use according to Claim 11, for preparing medicaments intended for treating chronic obstructive bronchitis, asthma, urinary incontinence, irritable bowel syndrome, Crohn's disease, ulcerative colitis, depression, anxiety, epilepsy, schizophrenia.

**13.** Medicament **characterized in that** it comprises a compound according to any one of Claims 1 to 4 or one of its pharmaceutically acceptable salts, solvates and/or hydrates.